# EUROPEAN PATENT APPLICATION

(11) **EP 4 697 005 A2**
(43) Date of publication of application: **18.02.2026**
(21) Application number: 25223703.7
(22) Date of filing: 09.09.2022
(51) Int. Cl.: G01N 1/36

(54) **TISSUE CUTTING SYSTEM AND METHOD**

(30) Priority: 10.09.2021 US 202163242542 P; 25.02.2022 US 202263313819 P
(62) Divisional of application: 22868097.1
(71) Applicant: Elephas Biosciences Corporation, Madison, Wisconsin 53717 (US)
(72) Inventor: Oliner, Jonathan Daniel, Madison,, 53717 (US); Cox-Muranami, Wesley Akira, Madison,, 53717 (US); Truong, Thieu Q., Madison,, 53717 (US); Fairbanks, Jonathan W., Madison,, 53717 (US); Tat, Allen, Madison,, 53717 (US); Fischer, Ryan E., Madison,, 53717 (US); Baltes, Christian R., Madison,, 53717 (US); Bakken, Todd J., Madison,, 53717 (US)
(74) Representative: Glawe, Delfs, Moll

(57) **Abstract**

The present invention relates to devices, systems, and methods for cutting tissues. In some embodiments, the devices, systems, and methods of the invention relate to cutting tissues into fragments that find use in tissue culture and drug testing applications.

## Description

The present application claims priority to United States Provisional Patent Application Serial Numbers 63/313,819, filed February 25, 2022 and 63/242,542, filed September 10, 2021, the entire discloses of which are hereby incorporated by reference in their entireties.

### FIELD

The present invention relates to devices, systems, and methods for cutting tissues. In some embodiments, the devices, systems, and methods of the invention relate to cutting tissues into fragments that find use in tissue culture and drug testing applications.

### BACKGROUND

Various diagnostic applications require tissue to be cut into thin sections. Conventional methods are limited by the speed of cutting and require one or more manual steps. As such, an unmet need exists to cut tissue into sections at high speed and precision, in an automated manner without causing significant mechanical damage to the tissue. It is also desirable to maintain maximum tissue viability for various downstream applications requiring live tissue, such as ex vivo drug response determination in various precision oncology applications.

### SUMMARY

One aspect of the present disclosure provides a tissue cutting system including a tissue holder with a hollow cavity. The tissue holder is configured to hold a tissue sample within the hollow cavity. The system further includes a first cutting component configured to cut a tissue sample contained within the hollow cavity of the tissue holder by scoring cuts in a first dimension and in a second dimension respectively, to produce a scored tissue sample. The system further includes a rotating device configured to cause a relative rotational movement between the tissue holder and the first cutting component, between a first relative orientation and a second relative orientation. In the first relative orientation, the first cutting component is configured to cut the tissue sample contained within the tissue holder in the first dimension. In the second relative orientation, the first cutting component is configured to cut the tissue sample contained within the tissue holder in the second dimension. The system further includes a second cutting component configured to cut the scored tissue sample in a third dimension to produce tissue fragments.

Another aspect of the present disclosure provides a tissue cutting system including a tissue holder with a hollow cavity, a proximal end, an opening at a distal end, and a sacrificial tissue-support positioned within the hollow cavity. The sacrificial tissue-support is configured to support a tissue sample. The sacrificial tissue-support is configured to be driven out of the hollow cavity to expose a portion of the tissue sample through the opening at the distal end. The system further includes a cutting component configured to cut the tissue sample to produce tissue fragments. The cutting component is configured to cut an exposed portion of the tissue sample by cutting through the sacrificial tissue-support. The system further includes a reservoir filled with a fluid material, and a portion of the tissue holder is submerged within the fluid material.

Another aspect of the present disclosure provides a tissue cutting system including a tissue holder configured to hold a tissue sample. The system further includes a cutting assembly including a mount, a first cutting component coupled to the mount, a second cutting component coupled to the mount, and an oscillator coupled to the mount. The oscillator is configured to move the first cutting component and the second cutting component. The first cutting component is configured to cut the tissue sample in a first dimension and a second dimension to produce a scored tissue sample. The second cutting component is configured to cut the scored tissue sample in a third dimension to produce tissue fragments. The system further includes a rotating device configured to cause a relative rotational movement between the tissue holder and the first cutting component between a first relative orientation and a second relative orientation. In the first relative orientation, the first cutting component is configured to cut a tissue sample contained within the tissue holder in the first dimension. In the second relative orientation, the first cutting component is configured to cut the tissue sample contained within the tissue holder in the second dimension. The system further includes a translation stage, wherein the tissue holder is coupled to the translation stage. The tissue holder translates with respect to the first cutting component and the second cutting component in response to activation of the translation stage.

Another aspect of the present disclosure provides a tissue cutting system for producing tissue fragments from a tissue sample, the tissue cutting system comprising a cutting component configured to cut the tissue sample into tissue fragments of a defined size and a reservoir configured to collect the tissue fragments. The system further includes a filter assembly attachable to the reservoir, wherein the filter assembly is configured to retain tissue fragments larger than the defined size.

Another aspect of the present disclosure provides a method of cutting a tissue sample using a tissue cutting system with a tissue holder including a hollow cavity, a first cutting component; and a second cutting component; the method comprising: preparing a tissue sample for cutting by positioning the tissue sample within the hollow cavity of the tissue holder; positioning the tissue holder in a first relative orientation; creating relative motion between the first cutting component and the tissue holder to make scoring cuts in the tissue sample in a first dimension; rotating the tissue holder by an angle to a second relative orientation; creating relative motion between the first cutting component and the tissue holder to make scoring cuts in the tissue sample in a second dimension, thereby producing a scored tissue sample; exposing a portion of the scored tissue sample through an opening at a distal end of the tissue holder; and moving an exposed portion of the scored tissue sample across the second cutting component, wherein the exposed portion of the scored tissue sample is cut in a third dimension to produce tissue fragments.

Another aspect of the present disclosure provides a method of preparing a live tissue sample for cutting comprising: providing a tissue holder including a hollow cavity, an opening at a distal end, and a sacrificial tissue-support; positioning the live tissue sample on a portion of the sacrificial tissue-support, wherein said portion of the sacrificial tissue-support is exposed out of the hollow cavity through the opening at the distal end of the tissue holder; coupling an encapsulant-reservoir to the opening at the distal end of the tissue holder, the encapsulant-reservoir includes an internal volume filled with an encapsulant precursor, wherein the sacrificial tissue-support supporting the live tissue sample extends into the internal volume of the encapsulant-reservoir filled with the encapsulant precursor; and retracting the sacrificial tissue-support supporting the live tissue sample into the hollow cavity of the tissue holder, wherein the encapsulant precursor is drawn into the hollow cavity along with the live tissue sample.

Another aspect of the present disclosure provides a kit for preparing a tissue sample for cutting, the kit comprising: a tissue holder comprising a hollow cavity, an opening at a distal end, and a sacrificial tissue-support. The sacrificial tissue-support is configured to support the tissue sample, and the sacrificial tissue-support is configured to be move relative to the hollow cavity. The sacrificial tissue-support is configured to be cut. The kit further includes an encapsulant-reservoir containing an encapsulant precursor. The encapsulant reservoir is configured to be detachably coupled to the opening at the distal end of the tissue holder thereby permitting the flow of the encapsulant precursor from the encapsulant-reservoir into the hollow cavity of the tissue holder.

Another aspect of the present disclosure provides a cutting assembly with a mount including a first surface and a first mount surface that intersects the first surface at a first angle. The first angle is 90 degrees. The mount further includes a second mount surface that intersects the first surface at a second angle, wherein the second angle is within a range of 1 degree to 20 degrees. The cutting assembly further includes a first cutting component coupled to the first mount; and a second cutting component coupled to the second mount surface.

Another aspect of the present disclosure provides a method of cutting a tissue sample using a tissue cutting system with a cutting component; the method comprising: moving the cutting component cyclically at a constant velocity in a first direction and at a constant velocity in a second direction opposite the first direction; moving the tissue sample toward the cutting component when the cutting component is moving at the constant velocity in the first direction or the second direction.

Other aspects of the disclosure will become apparent by consideration of the detailed description and accompanying drawings.

### BRIEF DESCRIPTION OF THE DRAWINGS

These and other features, aspects, and advantages of the present technology will become better understood with regards to the following drawings. The accompanying figures and examples are provided by way of illustration and not by way of limitation.
FIG. 1A is schematic side-view of a tissue cutting system.
FIG. 1B is schematic of a tissue cutting assembly including a filtration system, an oxygenation unit, an external chiller, and a control system.
FIG. 2A is a side view of a tissue cutting system.
FIG. 2B is a perspective view of the tissue cutting system of FIG. 2A.
FIG. 2C is a perspective view a tissue cutting system.
FIG. 2D is an enlarged view of a portion identified with a dotted box in FIG. 2C.
FIG. 2E is a perspective view of the tissue cutting system of FIG. 2C.
FIG. 3A is a side-view of a tissue cutting system.
FIG. 3B is another view of the tissue cutting system of FIG. 3A.
FIG. 3C is a perspective view of a portion of the tissue cutting system of FIG. 3A, illustrating a single assembly for both the cutting components.
FIG. 3D is a representation of the cutting components mounted on a single mount (e.g., cutting component holder), illustrating an angle δ between the planes of the cutting components.
FIG. 3E is a representation of the orientation of the first and second cutting components with respect to the tissue holder illustrating angles α1 and β.
FIG. 4A is a view of a cutting component illustrating various parts thereof.
FIG. 4B is a view of a cutting component illustrating various parts thereof.
FIG. 4C is a representation of a cutting component illustrating various parts thereof.
FIG. 4D is a side view of a cutting component with a double bevel.
FIG. 4E is a perspective view of an angle which the cutting components approach the tissue surface.
FIG. 5A is a perspective view of a tissue holder.
FIG. 5B is a perspective view of a tissue holder.
FIG. 5C is a side view of the tissue holders of FIGS. 5A and 5B, illustrating width (w), depth (d), and spacing (s) dimensions.
FIG. 6A is a perspective view of a tissue holder containing the tissue sample within the hollow cavity and supported on the sacrificial tissue-support.
FIG. 6B is a front view of the tissue holder with the tissue sample supported on the sacrificial tissue-support and exposed out of the hollow cavity.
FIG. 6C show various views of a sacrificial tissue-support comprising multiple grooves.
FIG. 7A illustrates a kit for preparing a tissue sample for cutting, showing the tissue sample supported on the sacrificial tissue-support.
FIG. 7B is a perspective view of the tissue sample supported on a sacrificial tissue-support and encased by an encapsulant. The tissue sample, supported on the sacrificial tissue-support and encased by the encapsulant, is shown in FIG. 7B to be exposed out of the hollow cavity of the tissue holder.
FIGS. 8A-8C are perspective views of a drive assembly, illustrating the tissue sample being driven out of the hollow cavity of the tissue holder.
FIG. 9A is a perspective view of a tissue holder in a first orientation relative to a cutting component and a second orientation relative to a cutting component.
FIG. 9B is a side view of a tissue cutting system with a tissue holder in a first orientation relative to a cutting component (left) and a second orientation relative to a cutting component (right).
FIG. 9C illustrates rotation of a cutting component between two orientations with respect to the tissue holder.
FIG. 10A is an exploded view of a reservoir and a nest.
FIG. 10B is a perspective view of the reservoir positioned within the nest of FIG. 10A.
FIG. 10C is a perspective cross-sectional view of FIG. 10B.
FIG. 10D is a cross-sectional view of FIG. 10B with arrows illustrating the direction of flow of a temperature-controlled liquid.
FIG. 10E is a perspective view of a nest.
FIG. 10F is a perspective cross-sectional view of the nest of FIG. 10E.
FIG. 10G is a perspective view of a tissue cutting system with a nest, an external heat-exchanger, and a controller.
FIG. 11 is a schematic of an oxygenation unit.
FIG. 12A is a schematic of a tissue cutting assembly showing a tissue cutting system, a filtration system, and components thereof.
FIG. 12B is a schematic of an in-line filtration system.
FIG. 12C is a schematic of an off-line filtration system.
FIG. 12D is a representation of operation of a filtration system.
FIG. 13 is a representation of steps to cut a tissue sample in a first dimension, wherein the first cutting component moves towards and away from a tissue holder (arrow a) containing the tissue sample in a first orientation. Successive cuts in a vertical dimension are enabled by vertical movement (arrow d) of the tissue holder.
FIG. 14 is a representation of steps to cut the tissue sample of FIG. 13 in a second dimension, wherein the first cutting component moves towards and away from the tissue holder (arrow a) containing the tissue sample in a second orientation. Successive cuts in a vertical dimension are enabled by vertical movement (arrow d) of the tissue holder.
FIG. 15 is a representation of steps to cut a tissue sample in a first dimension, wherein a tissue holder containing the tissue sample in a first orientation moves towards and away from a cutting component (arrow a1). Successive cuts in a vertical dimension are enabled by vertical movement (arrow d) of the tissue holder.
FIG. 16 is a representation of steps to cut the tissue sample of FIG. 15 in a second dimension, wherein the tissue holder containing the tissue sample in a second orientation moves towards and away from a cutting component (arrow a1). Successive cuts in a vertical dimension are enabled by vertical movement (arrow d) of the tissue holder.
FIG. 17 is a representation of steps to cut a tissue sample in a first dimension, wherein a tissue holder containing the tissue sample in a first orientation moves towards and away from a cutting component (arrow a1). Successive cuts in a horizontal dimension are enabled by horizontal movement (arrow e) of the tissue holder.
FIG. 18 is a representation of steps to cut the tissue sample of FIG. 17 in a second dimension, wherein the tissue holder containing the tissue sample in a second orientation moves towards and away from a cutting component (arrow a1). Successive cuts in a horizontal dimension are enabled by horizontal movement (arrow e) of the tissue holder.
FIG. 19 is a representation of steps to cut a tissue sample in a third dimension to create tissue fragments.
FIG. 20 is a representation of steps for preparing a tissue sample for cutting, illustrating various components involved in the process.
FIG. 21 is a perspective view of a cutting system.
FIG. 22 is a perspective view of a cutting assembly including a mount, a first cutting component, and a second cutting component.
FIG. 23 is a partial perspective cross-sectional view of the cutting assembly of FIG. 22.
FIG. 24 is a perspective view of the cutting assembly of FIG. 22.
FIG. 25 is an end view of the cutting assembly of FIG. 22.
FIG. 26 is an exploded view of a portion of the cutting system including a reservoir, a nest, a drive assembly, and a rotating device exploded from a translation assembly.
FIG. 27 is a side view of a tissue holder, a reservoir, and a nest.
FIG. 28 is a cross-sectional view of the reservoir, the nest, the drive assembly, and the rotating device of FIG. 26.
FIG. 29 is a perspective view of the drive assembly and the rotating device of FIG. 26.
FIG. 30 is a perspective view of the reservoir and the nest, with the tissue holder exploded from the reservoir.
FIG. 31A is a graph of sinusoidal motion for a cutting component and a motion stage.
FIG. 31B is a graph of motion for a cutting component and a motion stage.

Before any embodiments are explained in detail, it is to be understood that the invention is not limited in its application to the details of construction and the arrangement of components set forth in the following description or illustrated in the following drawings. The invention is capable of other embodiments and of being practiced or of being carried out in various ways.

### DETAILED DESCRIPTION

The singular forms "a" "an" and "the" include plural referents unless the context clearly dictates otherwise. Approximating language, as used herein throughout the specification and claims, may be applied to modify any quantitative representation that could permissibly vary without resulting in a change in the basic function to which it is related. Accordingly, a value modified by a term such as "about" is not to be limited to the precise value specified. Unless otherwise indicated, all numbers expressing quantities of ingredients, properties such as molecular weight, reaction conditions, so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least each numerical parameter should at least be construed in light of the number of reported significant digits and by applying ordinary rounding techniques.

The phrase "and/or," as used herein in the specification and in the claims, should be understood to mean "either or both" of the elements so conjoined, e.g., elements that are conjunctively present in some cases and disjunctively present in other cases.

Use of ordinal terms such as "first," "second," "third," etc., in the claims or specification to modify an element does not by itself connote any priority, precedence, or order of one claim element over another or the temporal order in which acts of a method are performed but are used merely as labels to distinguish one element having a certain name from another element having the same name.

The term "coupled," as used herein, is defined as "connected," although not necessarily directly, and not necessarily mechanically. The term coupled is to be understood to mean physically, magnetically, chemically, electrically, or otherwise coupled, connected or linked and does not exclude the presence of intermediate elements between the coupled elements absent specific contrary language.

The term "configured to" describes hardware, software or a combination of hardware and software that is adapted to, set up, arranged, commanded, altered, modified, built, composed, constructed, designed, or that has any combination of these characteristics to carry out a given function.

"Subject" as used herein is any mammalian or non-mammalian subject. In some embodiments, the subject is a human subject. In some embodiments, the subject is suspected of or diagnosed with cancer. The cancer can be any solid or hematologic malignancy. The cancer can be of any stage and/or grade. Non-limiting examples of cancer include cancers of head & neck, oral cavity, breast, ovary, uterus, gastro-intestinal, colorectal, pancreatic, prostate, brain and central nervous system, skin, thyroid, kidney, bladder, lung, liver, bone and other tissues.

"Tissue" or "tissue sample" as used interchangeably herein, is a biological material obtained from a subject. The tissue can be from any organ or site in the body of the subject. A tissue can be obtained from a subject by any approach known to a person skilled in the art. The tissue can be obtained by surgical resection, surgical biopsy, investigational biopsy or any other therapeutic or diagnostic procedure performed on a subject. In some embodiments, the tissue contains or is suspected to contain tumor cells. The terms tumor cells, cancerous cells, and malignant cells have been used interchangeably. In some embodiments, the tissue is a tumor tissue. In some embodiments, the tissue is obtained from any organ or site in the body of the subject where a cancer has originated or where the cancer has metastasized to. In some embodiments, the tissue may also contain immune cells, stromal cells etc. While the tissue can be in any form (such as frozen or fixed), in preferred embodiments, the tissue is a live, fresh tissue. In some embodiments, the tissue has not been subjected to any tissue fixation techniques known to a person of ordinary skill in the art (such as formalin treatment) or not been stored under any condition or for any duration of time to significantly reduce the number of viable cells.

Tissue fragments are fragments of the tissue sample that have detached from the tissue sample, wherein the fragments are obtained by cutting the tissue in one or more dimensions. In some embodiments, the tissue fragments are obtained by cutting the tissue sample in all three dimensions, such as a first dimension, a second dimension, and a third dimension. In some embodiments, (such as in the case of a biopsy tissue sample) where the tissue sample already has the desired sizes in two dimensions, tissue fragments can be produced by cutting the tissue sample in only one dimension. The tissue fragments can be of various shapes, with non-limiting examples of shapes including cubes, square cuboids, rectangular cuboids, parallelogram prisms and the like. In some embodiments, the tissue fragments are substantially cubical in shape. In some embodiments, the size of each tissue fragment is equal to or less than 1000 µm (such as 1000 µm, 500 µm, 450 µm, 400 µm, 350 µm, 300 µm, 250 µm, 200 µm, 100 µm or 50 µm) in at least one dimension. In some embodiments, the size of each tissue fragment is between 50 µm and 1000 µm in at least one dimension. In some embodiments, the size of each tissue fragment is between 100 µm and 500 µm in at least one dimension. In some embodiments, the size of each tissue fragment is between 150 µm and 350 µm in at least one dimension. In some embodiments, the size of each tissue fragment is between 50 µm and 500 µm (such as 50 µm, 100 µm, 150 µm, 200 µm, 250 µm, 300 µm, 350 µm, 400 µm, 450 µm or 500 µm) in at least two dimensions. In some embodiments, the size of each tissue fragment is between 100 µm and 350 µm in at least two dimensions. In some embodiments, the size of each tissue fragment is between 50 µm and 500 µm in all three dimensions. In some embodiments, the size of each tissue fragment is between 100 µm and 350 µm in all three dimensions. In some embodiments, each tissue fragment is between 300 µm and 350 µm in two dimensions and between 100 µm and 150 µm in a third dimension. In some embodiments, the tissue fragments are uniform in size. As used herein, uniform means substantially uniform, wherein the size of the tissue fragments are within ±30% of one another, in at least one dimension. In some embodiments, the tissue fragments are live tissue fragments, wherein the cutting processes did not substantially reduce the number of viable cells that were present in the tissue sample. In some embodiments, the tissue fragments are live tissue fragments, such that one or more functional assays can be performed on the tissue fragments. A specified size is the desired size of a tissue fragment in one or more dimensions. The specified size can be user-defined or pre-defined depending on tissue type and/or end application. According to one or more embodiments, the tissue cutting system cuts the tissue sample into tissue fragments of a specified size. The size of the tissue fragments is specified in one or more dimensions. In some embodiments, the tissue cutting system cuts the tissue into tissue fragments as per sizes specified in all three dimensions. As used herein, a tissue fragment of a specified size does not necessarily imply that the tissue fragment has the same size in all dimensions. For example, the tissue fragment of a specified size can have the same size in all three dimensions (such as 300 µm × 300 µm × 300 µm), it can have the same size in two dimensions and a different size in the third dimension (such as 300 µm × 300 µm × 100 µm), or it can have different sizes in all three dimensions. Tissue fragments that are cut in sizes greater than or less than the specified size (such as in one, two or all three dimensions), depending on the end application, are unwanted tissue fragments. In some embodiments, tissue fragments within ±50% of the specified size (in one or more dimensions) can still be usable or are desired tissue fragments. For example if the specified size is 300 µm × 300 µm × 300 µm, tissue fragments with a size of 450 µm in one or more dimensions might still be within the range of specified size (hence desired tissue fragments), however, tissue fragments with size exceeding 450 µm in one or more dimensions might be outside the range of the specified size and hence are unwanted tissue fragments. The size that is acceptable within the range of specified size may be user defined based on the application.

A cutting plane is a plane at which the tissue sample is cut. A cutting event is the process of cutting the tissue at one cutting plane. In some embodiments, the dimensions at which a tissue is cut (that is, the first dimension, the second dimension and the third dimension) are mutually perpendicular to one another. "Successive cutting planes" as used herein refers to a plurality of substantially parallel cutting planes in one dimension.

A cutting process is the process of cutting the tissue sample at a plurality of cutting planes in at least one dimension. In some embodiments, a cutting process comprises multiple cutting events. A first cutting process is the process of cutting the tissue at multiple cutting planes in a first dimension, a second cutting process is the process of cutting the tissue at multiple cutting planes in a second dimension, and a third cutting process is the process of cutting the tissue at multiple cutting planes in a third dimension. In some embodiments, the first, the second, and the third cutting processes are sequential. In some embodiments, the first and the second cutting processes are scoring processes, where the tissue sample is cut at successive planes in the first and the second dimensions respectively, but tissue fragments (*i.e.,* fragments that detach from the tissue sample) are not produced. As to be understood, a "scoring cut" is a cut that cuts a tissue sample but doesn't produce tissue fragments that detach from the tissue sample. As to be understood by a person skilled in the art, a scoring cut is a shallow cut that does not penetrate the entire depth of the tissue sample to produce a fragment that detaches from the tissue sample. A "scored tissue sample" is a tissue sample or a portion thereof that has been cut by scoring cuts in the first and the second dimensions, but tissue fragments (*i.e.,* fragments that detach from the tissue sample) are not produced. In some embodiments, tissue fragments are produced only after the tissue sample is cut in all three dimensions. A "slicing cut" is a cut that produces tissue fragments which detach from the tissue sample. In some embodiments, the cut in the third dimension by the second cutting component (after scoring cuts in the first and the second dimension) is a slicing cut. In some embodiments, especially for biopsy samples (where the tissue sample already has the desired sizes in two dimensions), the cuts in a single dimension produces tissue fragments that detach from the tissue sample and hence is a slicing cut.

A cutting cycle is the sequential or concurrent occurrence of a first cutting process, a second cutting process and a third cutting process to produce tissue fragments. In some embodiments, a cutting cycle is the sequential occurrence of a first cutting process, followed by a second cutting process and finally followed by a third cutting process, to produce tissue fragments. In some embodiments, one cutting cycle cuts only a portion of the tissue sample into tissue fragments. In some embodiments, multiple cutting cycles are required to cut the entire tissue sample into tissue fragments.

A cutting component is any object configured to cut a tissue precisely. The cutting component is configured to cut the tissue precisely at cutting plane in a given dimension with minimal damage to the tissue at the cutting plane or at adjoining regions thereof. According to some embodiments, a cutting plane is a plane at which a cutting component cuts the tissue. Non-limiting examples of cutting components include blades, wires, or scalpels. In some embodiments, the cutting component is a blade.

As used herein, the term "processor" (e.g., a microprocessor, a microcontroller, a controller, a processing unit, or other suitable programmable device) can include, among other things, a control unit, an arithmetic logic unit ("ALC"), and a plurality of registers, and can be implemented using a known computer architecture (e.g., a modified Harvard architecture, a von Neumann architecture, etc.). In some embodiments the processor is a microprocessor that can be configured to communicate in a stand-alone and/or a distributed environment, and can be configured to communicate via wired or wireless communications with other processors, where such one or more processor can be configured to operate on one or more processor-controlled devices that can be similar or different devices.

As used herein, the term "memory" is any memory storage and is a non-transitory computer readable medium. The memory can include, for example, a program storage area and the data storage area. The program storage area and the data storage area can include combinations of different types of memory, such as a ROM, a RAM (e.g., DRAM, SDRAM, etc.), EEPROM, flash memory, a hard disk, a SD card, or other suitable magnetic, optical, physical, or electronic memory devices. The processor can be connected to the memory and execute software instructions that are capable of being stored in a RAM of the memory (e.g., during execution), a ROM of the memory (e.g., on a generally permanent bases), or another non-transitory computer readable medium such as another memory or a disc. In some embodiments, the memory includes one or more processor-readable and accessible memory elements and/or components that can be internal to the processor-controlled device, external to the processor-controlled device, and can be accessed via a wired or wireless network. Software included in the implementation of the methods disclosed herein can be stored in the memory. The software includes, for example, firmware, one or more applications, program data, filters, rules, one or more program modules, and other executable instructions. For example, the processor can be configured to retrieve from the memory and execute, among other things, instructions related to the processes and methods described herein.

As used herein, the term "network" generally refers to any suitable electronic network including, but not limited to, a wide area network ("WAN") (e.g., a TCP/IP based network), a local area network ("LAN"), a neighborhood area network ("NAN"), a home area network ("HAN"), or personal area network ("PAN") employing any of a variety of communications protocols, such as Wi-Fi, Bluetooth, ZigBee, etc. In some embodiments, the network is a cellular network, such as, for example, a Global System for Mobile Communications ("GSM") network, a General Packet Radio Service ("GPRS") network, an Evolution-Data Optimized ("EV-DO") network, an Enhanced Data Rates for GSM Evolution ("EDGE") network, a 3GSM network, a 4GSM network, a 5G New Radio, a Digital Enhanced Cordless Telecommunications ("DECT") network, a digital AMPS ("IS-136/TDMA") network, or an Integrated Digital Enhanced Network ("iDEN") network, etc. In some embodiments, systems comprise a computer and/or data storage provided virtually (e.g., as a cloud computing resource). In particular embodiments, the technology comprises use of cloud computing to provide a virtual computer system that comprises the components and/or performs the functions of a computer as described herein. Thus, in some embodiments, cloud computing provides infrastructure, applications, and software as described herein through a network and/or over the internet. In some embodiments, computing resources (e.g., data analysis, calculation, data storage, application programs, file storage, etc.) are remotely provided over a network (e.g., the internet).

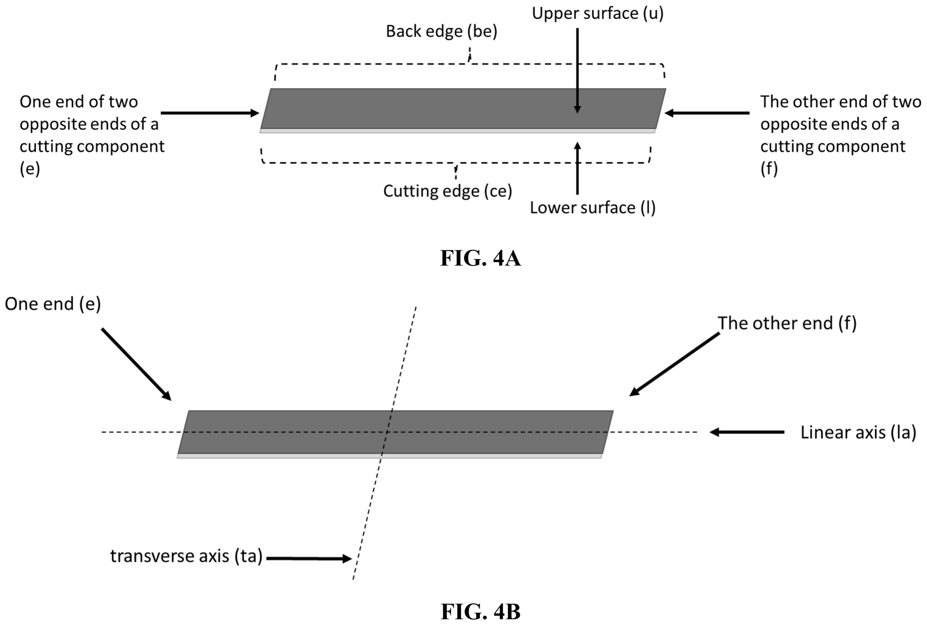

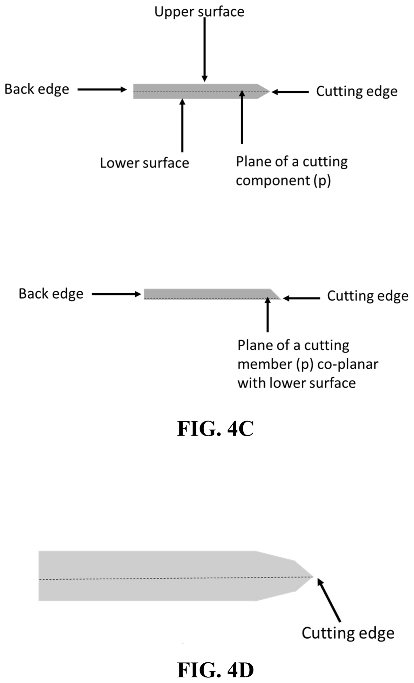

In some embodiments, as represented by FIGS. 4A, 4B, and 4C, a cutting component includes an upper surface (u), a lower surface (1), two opposite ends (e and f), a cutting edge (ce) extending between the two opposite ends (e and f), and a back edge (be), opposite to the cutting edge (ce), also extending between the two opposite ends (e and f). In some embodiments, at least a portion of the upper and the lower surfaces are parallel to each other. A plane of the cutting component (p) refers to a plane extending from the cutting edge to the back edge. In some embodiments, the plane of the cutting component is a plane equidistant from the upper surface and the lower surface. In some embodiments, the plane of the cutting component is co-planar with at least one of the upper or the lower surface. In embodiments, the cutting component comprises double bevel (such as shown in FIG. 4D). In some embodiments, the cutting component is composed of materials such as, but not limited to, stainless steel, tungsten carbide, sapphire, or zirconium.

With reference to FIG. 4B, a linear axis (la) of a cutting component is an axis extending from one end of the cutting component to the other end. The linear axis is parallel to the cutting edge. In some embodiments, the linear axis is the longest axis of the cutting component that is parallel to the cutting edge. A transverse axis (ta) is an axis perpendicular to the linear axis. The transverse axis is parallel to the plane of the cutting component (p).

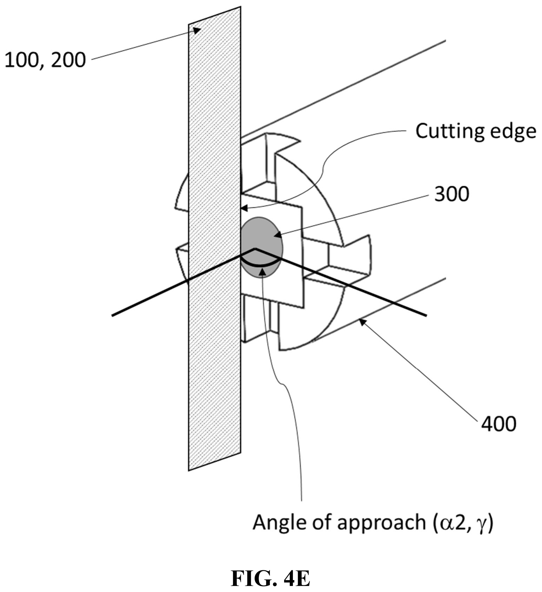

With reference to FIG. 4e, an angle of approach is the angle that the plane of a cutting component (shown as the area shaded with parallel lines) makes with the surface of the tissue sample 300 (shown as area shaded in grey). In some embodiments, it is the angle at which the cutting component (100 or 200) approaches the tissue sample 300. In some embodiments, the angle of approach of the first cutting component 100 is denoted by α2 and the angle of approach of the second cutting component 200 is denoted by γ. The surface of the tissue sample is the face of the tissue sample which is exposed to the cutting components, and which makes the first contact with the cutting components.

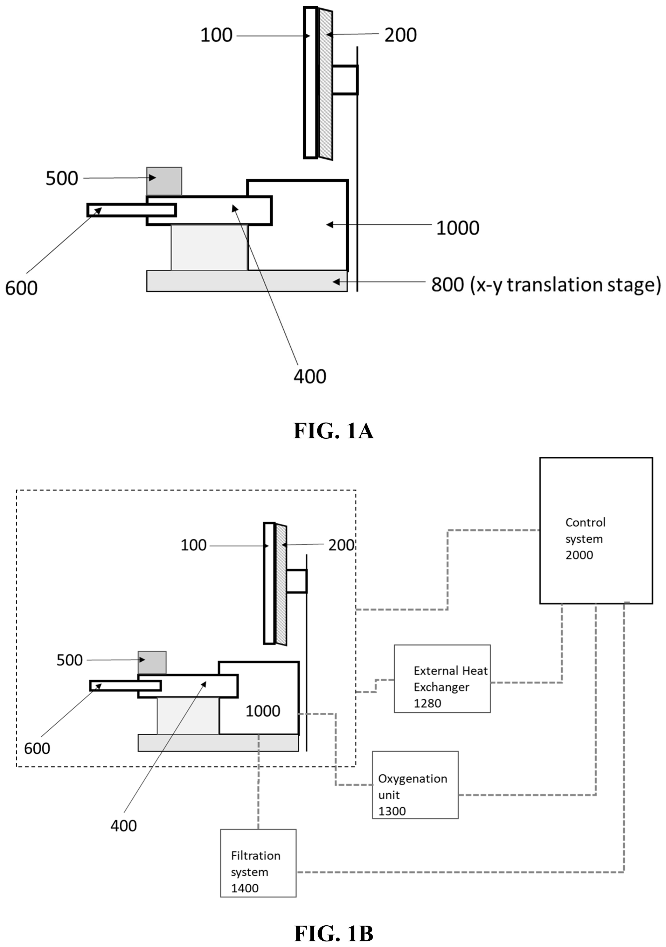

In some embodiments, a first cutting component 100 (FIG. 1A) is configured to cut the tissue sample in a first dimension and in a second dimension. In some embodiments, the first cutting component is part of a first cutting assembly. In some embodiments, the first cutting assembly comprises a first cutting component holder (e.g., mount) configured to hold the first cutting component. In some embodiments, the first cutting component is configured to be moved towards and away from the tissue sample. In some embodiments, the translational movement of the first cutting component towards and away from the tissue sample or the translational movement of the tissue holder containing the tissue sample towards and away from the first cutting component is referred to as a scoring motion. A scoring motion is one that enables a scoring cut. In some embodiments, the first cutting assembly comprises a first translational assembly configured to drive the scoring motion of the first cutting component towards and away from the tissue holder. In some embodiments, the first cutting component is configured to be oscillated, such as about its linear axis or its transverse axis. In some embodiments, the first cutting assembly comprises a first oscillator configured to oscillate the first cutting component about its linear axis. In some embodiments, the oscillation motion and the motion towards and away from the tissue sample (or the scoring motion) of the first cutting component are driven by a voice coil actuator. This allows for driving both types of motion with one single unit, hence offering the advantage of a smaller device footprint. Also, the frequency and speed can be adjusted during the process, while the first cutting component is in motion. This allows for improved control over the speed and/or frequency of the first cutting component during the cutting process.

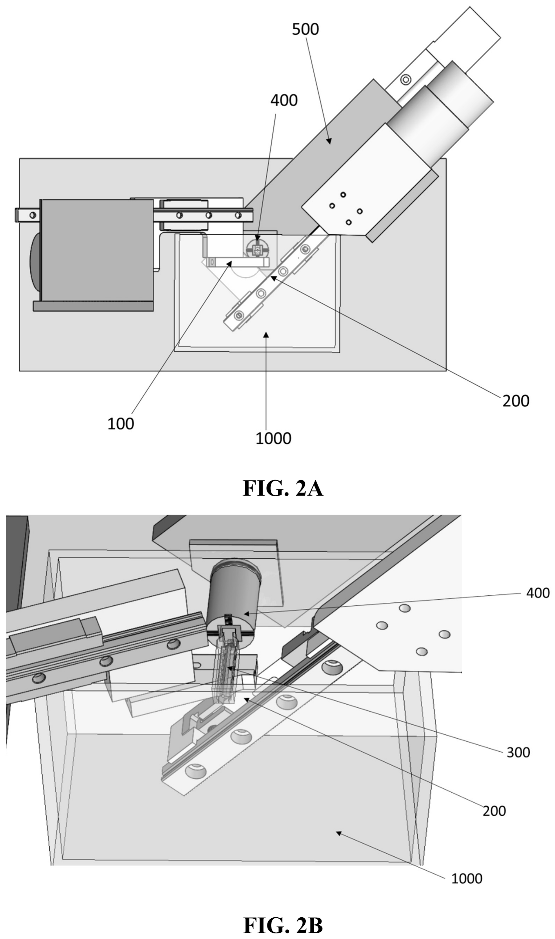

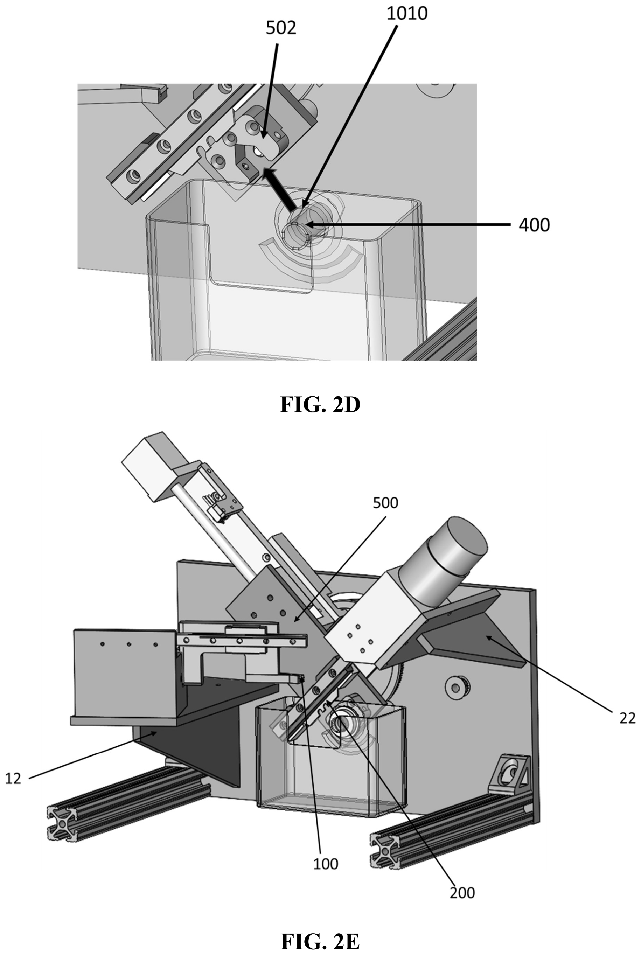

With reference to FIG. 2E, in some embodiments, the first cutting assembly further comprises a first support member 12 to which the first cutting component 100 is coupled. In some embodiments, the first support member 12 is configured to support the first cutting component 100 and position the first cutting component 100 with respect to the other components and the sub-components of the tissue cutting system.

In some embodiments, a second cutting component 200 (FIG. 1A) is configured to cut the tissue sample in a third dimension. In some embodiments, the second cutting component is part of a second cutting assembly. In some embodiments, the second cutting assembly comprises a second cutting component holder (e.g., mount) configured to hold the second cutting component. In some embodiments, the second cutting assembly further comprises a second oscillator configured to oscillate the second cutting component about its linear axis.

With reference to FIG. 2E, in some embodiment, the second cutting assembly further comprises a second support member 22 to which the second cutting component 200 is coupled. In some embodiments, the second support member 22 is configured to support the second cutting component 200 and position the second cutting component 200 with respect to the other components and the sub-components of the tissue cutting system.

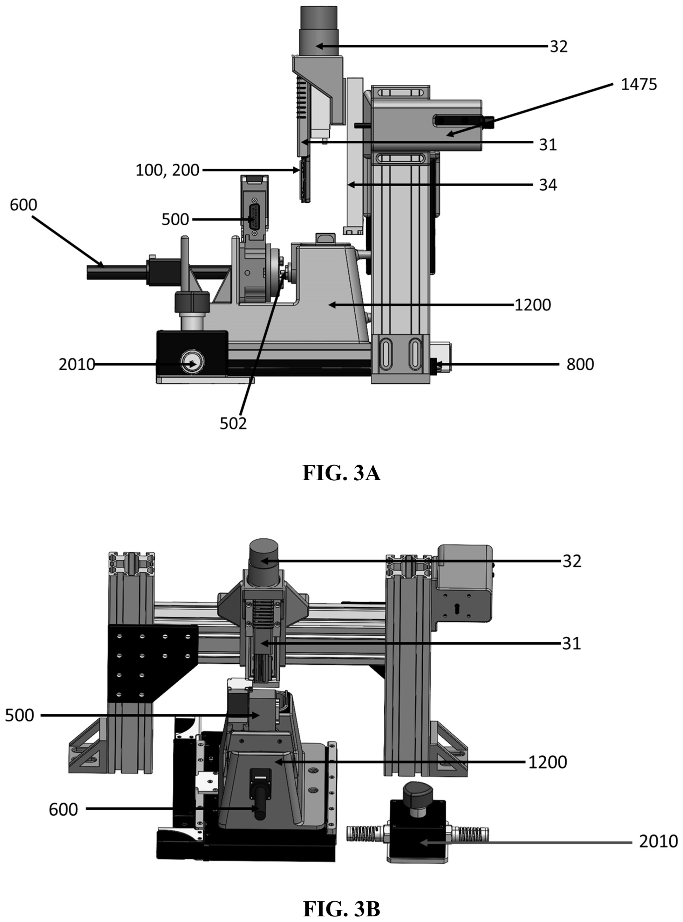

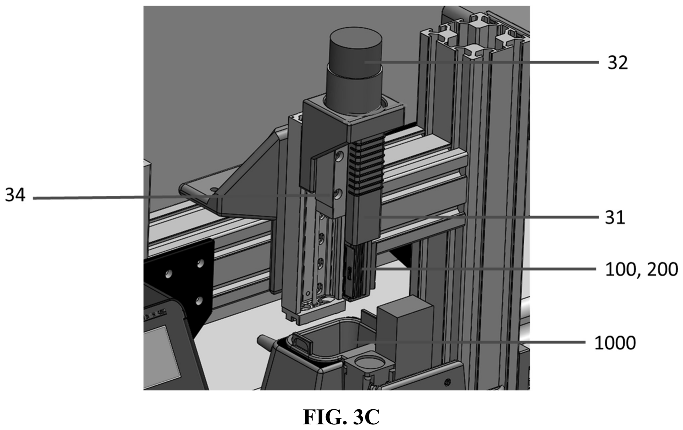

With reference to FIGS. 3A, 3B, and 3C, the first cutting component 100 and the second cutting component 200 are both part of a single cutting assembly. In some embodiments, the first cutting component and the second cutting component are mounted on a single cutting component holder 31 (e.g., a single mount). The cutting components, mounted on the single cutting component holder 31, are positioned at different angles (with respect to the tissue sample) to enable scoring cuts by the first cutting component 100, and slicing cuts by the second cutting component 200, without needing to change the orientation of the cutting components or the tissue sample with respect to one another, during operation.

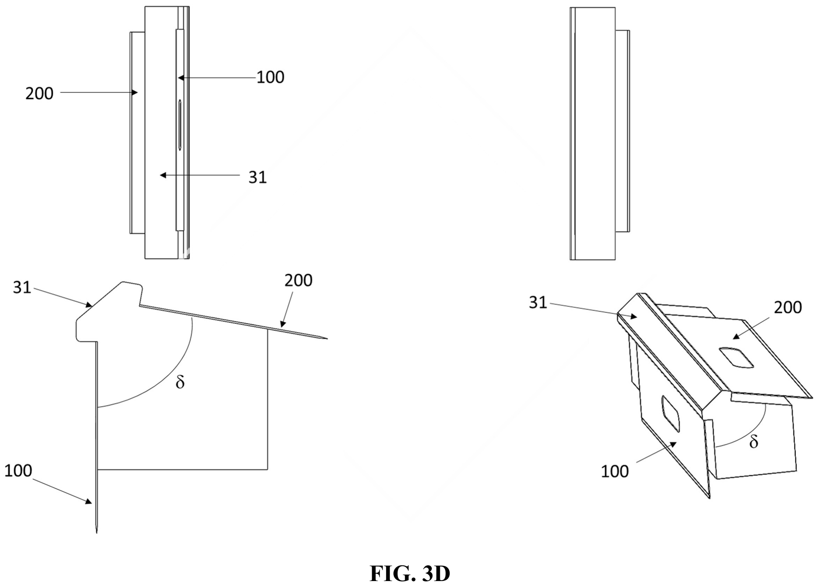

With reference to FIG. 3D, the cutting components 100, 200 are oriented at an angle δ with respect to each other when mounted on the cutting component holder 31.

In some embodiments, an oscillator is configured to oscillate a cutting component (e.g., the first and/or the second cutting component) about one or more axes of the cutting components (e.g., a linear axis and/or the transverse axis). In some embodiments, an oscillator includes a motor e.g., a piezoelectric motor, a set of piezoelectric motors, an electric motor, or the like. The oscillator controls the frequency and the amplitude of oscillations of the cutting components. In some embodiments, the cutting component is configured to be oscillated at frequencies within a range of approximately 20 to approximately 200 Hz. In some embodiments, the cutting component is configured to be oscillated at frequencies within a range of approximately 50 to approximately 200 Hz. In some embodiments, the cutting component is configured to be oscillated at frequencies within a range of approximately 120 to approximately 200 Hz. In some embodiments, the oscillator comprises a voice coil actuator. In some embodiments, a voice coil actuator drives the oscillation of the cutting components. In some embodiments, a single voice coil actuator drives the oscillation of both the first and second cutting components.

With reference to FIG. 3A-3C, in some embodiments, a single oscillator 32 is connected to a linear rail system 34. A single mount 31 (e.g., cutting component holder) is coupled to the linear rail system 34. The two cutting components 100, 200 are coupled to the mount 31 and are oriented at different angles with respect to the surface of the tissue sample. In some embodiments, as shown in FIG. 3D, the plane of the first cutting component 100 is at an angle δ with respect to the plane of the second cutting component 200. In some embodiments, the oscillator 32 is configured to change the frequency and amplitude of oscillation of the first and the second cutting components between the scoring and the slicing cuts. For example, during the scoring cuts by the first cutting component 100, the oscillator 32 oscillates the first and the second cutting components 100, 200 at a first specified frequency and amplitude. After the scoring cuts, the oscillator 32 oscillates the first and the second cutting components 100, 200 at a second specified frequency and amplitude, to enable slicing cuts by the second cutting component 200.

In some embodiments, the tissue sample is moved from one cutting component to another at specific rates and motion patterns to create the scoring cuts with one cutting component 100 and slicing cuts with the other 200. In some embodiments, the only motion of the cutting components during the cutting operation is the oscillation motion driven by the oscillator.

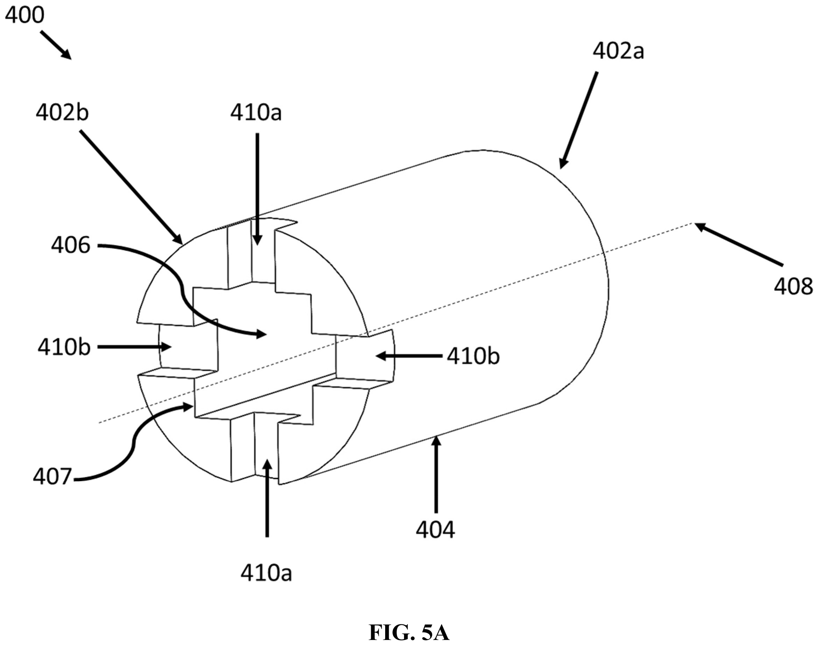

With reference to FIG. 5A, a tissue holder 400 is configured to support the tissue sample 300. The tissue holder 400 comprises a proximal end 402a, a distal end 402b, a sidewall 404, a hollow cavity 406 defined by the sidewall 404, and at least one opening 407 at the distal end 402b. The tissue holder 400 is configured to hold the tissue sample 300 within the hollow cavity 406. In the illustrated embodiment, a longitudinal axis 408 of the tissue holder 400 is an axis extending between the proximal end 402a and the distal end 402b. In the illustrated embodiment, the tissue holder 400 has a circular cross-sectional shape. In other embodiments, the tissue holder has a cross-sectional shape that is elliptical, square, rectangular and the like. In some embodiments, the tissue holder 400 is configured to stably hold the tissue sample within the hollow cavity during the cutting processes. In some embodiments, the tissue holder 400 is composed of a rigid material, such as steel. Advantageously, the tissue holder 400 minimizes any movement or wobble of the tissue sample 300 during the cutting processes.

With continued reference to FIG. 5A, the tissue holder 400 includes a first slit 410a and a second slit 410b formed in the sidewall 404. In other words, the slits 410a, 410b are gaps, notches, grooves, etc. in the sidewall 404 of the tissue holder 400. A cutting component can pass into the interior of the hollow cavity 406 through the slit 410a or 410b and cut the tissue sample 300, while the tissue sample 300 is contained within the hollow cavity 406. In the illustrated embodiment, the slit 410a extends from one portion of the sidewall 404 to an opposite portion of the sidewall 404. In the illustrated embodiment, the first slit 410a and the second slit 410b are oriented at approximately 90° angle with respect to each other.

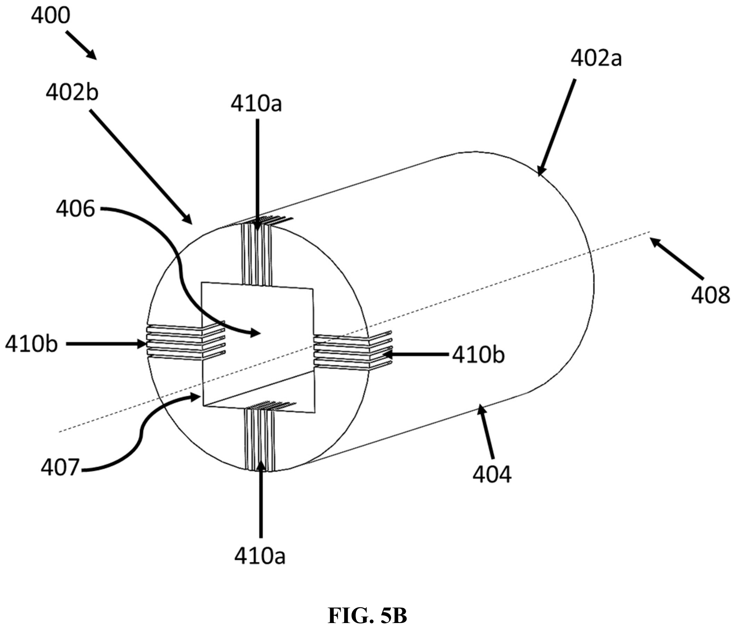

With reference to FIG. 5B, a tissue holder 400 is illustrated with a sidewall 404 having a plurality of first slits 410a and a plurality of second slits 410b. In the illustrated embodiment, the sidewall 404 includes a plurality of first slits 410a and a plurality of second slits 410b, wherein each slit in the plurality of first slits is at 90° angle with respect to each slit in the plurality of second slits. In some embodiments, the slit or the plurality of slits are located at or near the distal end 402b of the tissue holder.

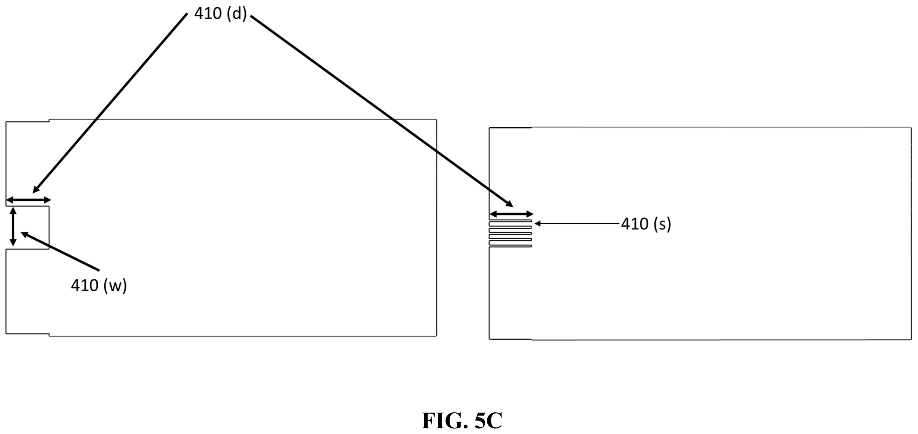

With reference to FIG. 5C, the width of a slit is represented by 410(w). In some embodiments, 410(w) is within a range of approximately 1 mm and approximately 10 mm. The spacing between adjacent slits in the plurality of slits is represented by 410(s). In some embodiments, 410(s) is the distance from the edge of one slit to the nearest edge of an adjacent slit. In some embodiments, the spacing 410(s) is between about 300 µm and about 2000 µm. In other words, two adjacent slits of the plurality of slits are positioned apart within a range of approximately 300 µm and approximately 2000. The depth of the slit is represented by 410(d). In some embodiments, the depth of the slit is within a range of approximately 50 µm and approximately 1000 µm.

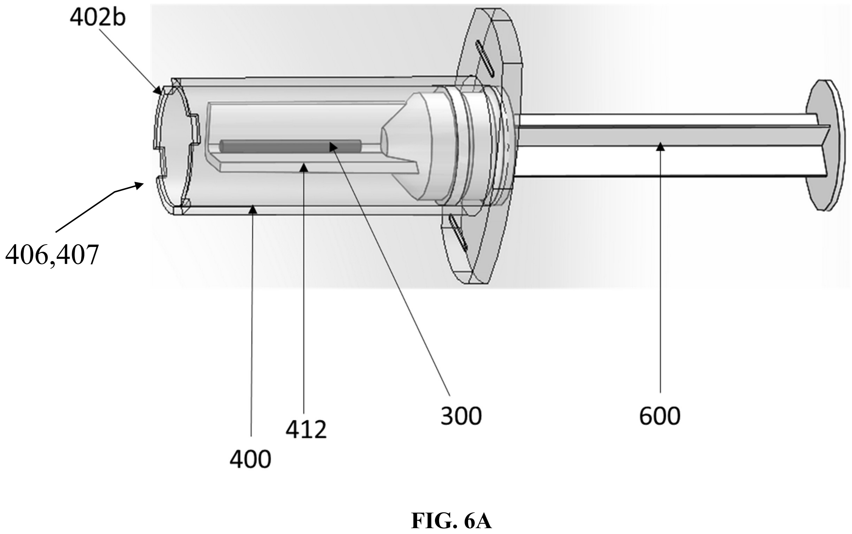

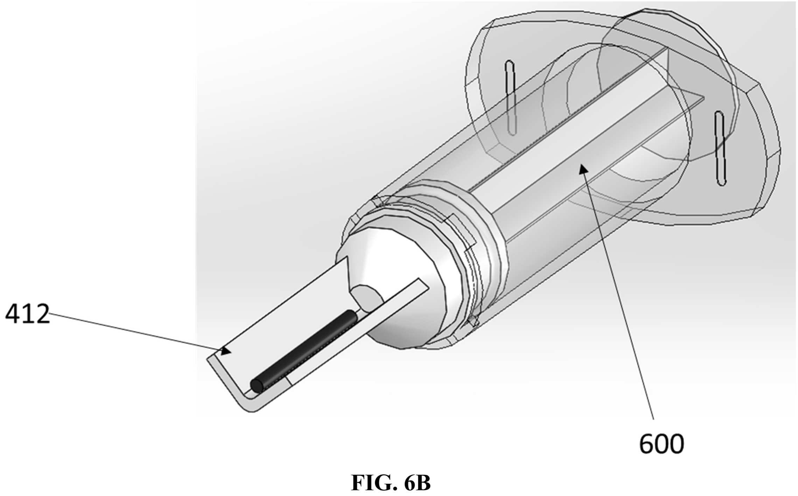

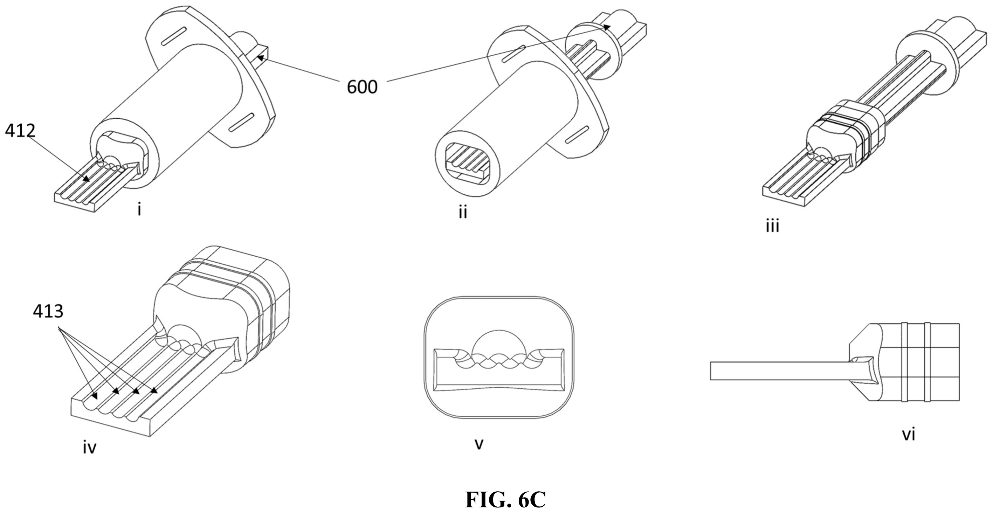

With reference to FIG. 6A and 6B, in some embodiments, the tissue holder 400 further includes a sacrificial tissue-support 412 (e.g., a scaffold). In some embodiments, the sacrificial tissue-support is an integral part of the tissue holder. In some embodiments, the sacrificial tissue-support is detachable. The sacrificial tissue-support 412 is configured to support the tissue sample 300. The sacrificial tissue-support 412 is configured to be driven out of the hollow cavity 406 of the tissue holder 400 through the opening 407 at the distal end 402b (FIG. 6B) and retracted back into the hollow cavity 406. In some embodiments, the sacrificial tissue-support 412 is configured to be driven in and out of the hollow cavity 406 by a drive assembly 600 coupled to the sacrificial tissue-support 412. In some embodiments, the drive assembly 600 is a manual plunger. In other embodiments, the drive assembly 600 is an electronically controlled linear actuator.

In some embodiments, the sacrificial tissue-support 412 comprises a non-flat surface. Various types of non-flat surfaces can be envisaged, such as a concave surface, a U-shaped surface, an angular V-shaped surface and the like. In some embodiments, the sacrificial tissue-support comprises a surface that is wide at one end and tapers into a groove at the other end. The groove is configured to receive and support the tissue sample 300. In some embodiments, the sacrificial tissue-support has a V-shaped surface. The tissue sample is supported on the groove of the V-shaped surface of the sacrificial tissue-support. The non-flat shape of the sacrificial tissue-support ensures that the tissue sample supported on the groove can be positioned as close as possible to the center of the tissue holder. The sacrificial tissue support can be of various form factors that best suits the input tissue. For example, biopsies with long cylinder-like shapes need a differently shaped support from excisions that are amorphous blobs.

With reference to FIG. 6C, the sacrificial tissue-support 412 in some embodiments includes a plurality of grooves 413, wherein one tissue sample is supported on one groove. In other words, each of the plurality of grooves 413 is configured to receive a separate tissue sample. The plurality of grooves 413 enables simultaneous cutting of multiple tissue samples. FIG. 6C(i) illustrate the sacrificial tissue support 412 exposed out of the hollow cavity of the tissue holder 400 and FIG. 6C(ii) illustrates the sacrificial tissue support 412 retracted into the tissue holder with the help of the drive assembly 600. In some embodiments, the dimensions of the grooves 413 can be tailored to match the dimensions of the tissue samples. In some embodiments, the dimension of the grooves is slightly larger than the dimension of the tissue sample to allow enough space for the tissue sample to be completely encased by an encapsulant. In some embodiments, an adhesive may be used to secure the tissue sample on the sacrificial tissue-support. In some embodiments the adhesive is applied on the sacrificial tissue-support to promote adhesion of the tissue sample.

The sacrificial tissue-support 412 is composed of a material that can be cut with a cutting component (such as a sacrificial material). Non-limiting examples of materials of the sacrificial tissue-support include wax, silicone (such as polydimethylsiloxane (PDMS)), polycarbonates, polypropylenes, polyurethanes, cyclo-olefin polymers or combinations thereof. In some embodiments, the material of the sacrificial tissue support 412 is biocompatible and non-toxic to avoid damaging or altering the tissue properties. In some embodiments, the material of the sacrificial tissue-support is such that the sacrificial tissue-support does not bend or buckle significantly under the pressure of a cutting component, thereby minimizing any mechanical damages caused to the tissue during cutting. The sacrificial tissue-support 412 advantageously serves to mitigate any shape deviations, relative to the intended dimensions, that can be caused to the tissue or tissue fragments during cutting.

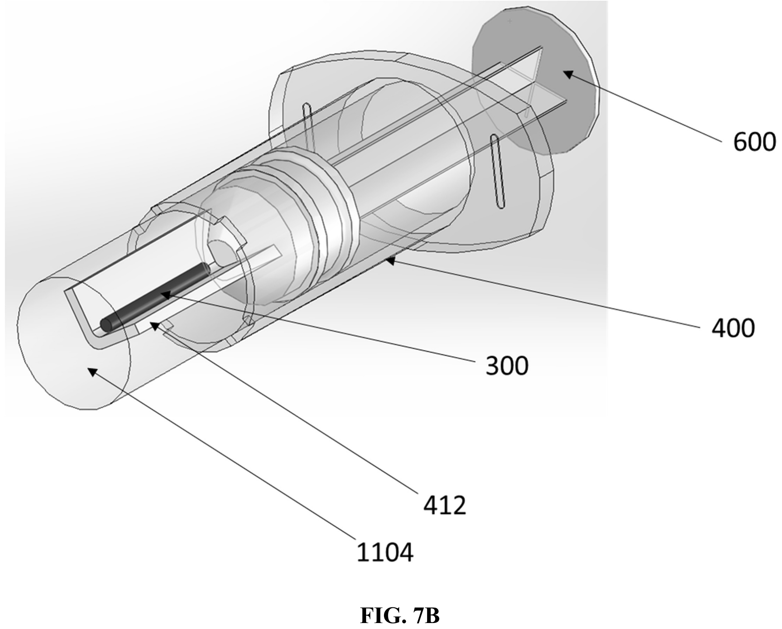

With reference to FIG. 7B, in some embodiments, an encapsulant 1104 secures the tissue sample 300 in position within the tissue holder 400 during the cutting process. In some embodiments, the encapsulant is a gel, wherein the encapsulant precursor can be caused to gel under gelation conditions. The encapsulant used herein can be a natural, a synthetic or a semi-synthetic hydrogel material. In some embodiments, the encapsulant is low melting agarose gel. In some embodiments, the encapsulant is alginate gel. In some embodiments, the encapsulant precursor is an alginate solution in the liquid state. In some embodiments, the encapsulants can be cut with the cutting component. In some embodiments, the encapsulants provide sufficient stability, wherein the tissue sample does not significantly buckle or deform under the pressure of the cutting component. In some embodiments, the encapsulant is composed of a biocompatible material that does not sufficiently affect the viability or otherwise alter the biological properties of the tissue sample.

As used herein, an encapsulant precursor is a component that forms the encapsulant in the gel state under suitable conditions of gelation. The encapsulant precursor can be in any physical form such as in liquid or in solid form. In some embodiments, the encapsulant is formed by a covalent cross-linking of the encapsulant precursors, while in some other embodiments the encapsulant is formed by a physical aggregation of the encapsulant precursors. In some embodiments, depending upon the tissue type, the percentages of the encapsulant precursors and/or gelation conditions can be varied to obtain encapsulants of varying mechanical stiffness.

The terms "gelling" and "gelation" as used herein, means physical aggregation and/or chemical/covalent cross-linking (or polymerization) of encapsulant precursors to form the encapsulant. Gelation or gelling conditions are conditions that cause physical aggregation and/or chemical/covalent cross-linking of the encapsulant precursors to form the encapsulant. Non-limiting gelling conditions are temperature change or photo-irradiation. In some embodiments, gelling or gelation conditions cause sol to gel transition of an encapsulant precursor (in the sol state) to the encapsulant (in the gel state). The gelation conditions preferably do not cause substantial damage to the tissue sample. In some embodiments, gelation happens at a low temperature, within a short time, and preferably under mild chemical conditions.

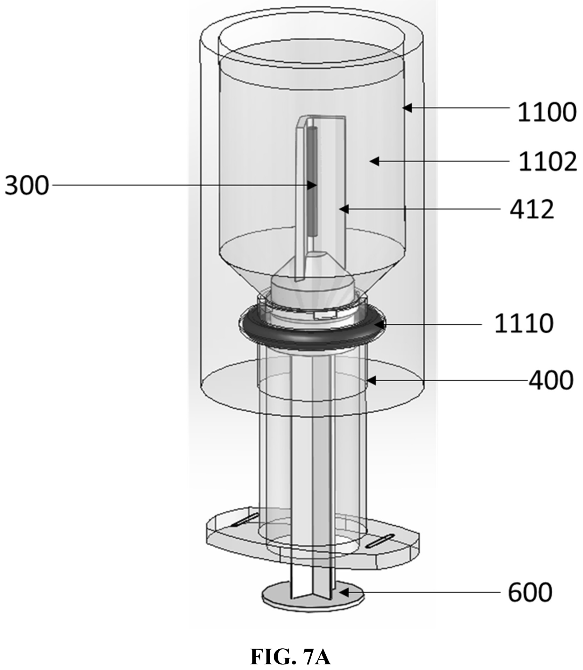

With reference to FIG. 7A, an encapsulant-reservoir 1100 is a container configured to hold the encapsulant precursors (such as in the liquid state). The encapsulants precursors are contained within an internal volume 1102 of the encapsulant-reservoir 1100. In some embodiments, the encapsulant-reservoir 1100 containing the encapsulant precursors is configured to be coupled to the tissue holder 400. In some embodiments, the encapsulant-reservoir is configured to be coupled to the tissue holder, wherein the encapsulant-reservoir forms an interface with the opening 407 at the distal end 402b of the tissue holder 400. In some embodiments, the encapsulant-reservoir containing the encapsulant precursors is coupled to the tissue holder with the help of an O-ring 1110 that creates a substantially liquid-impermeable seal at the interface preventing any leakage of the encapsulant precursors. In some embodiments, the interface allows the flow of the encapsulant precursor from the internal volume of the encapsulant-reservoir into the hollow cavity of the tissue holder.

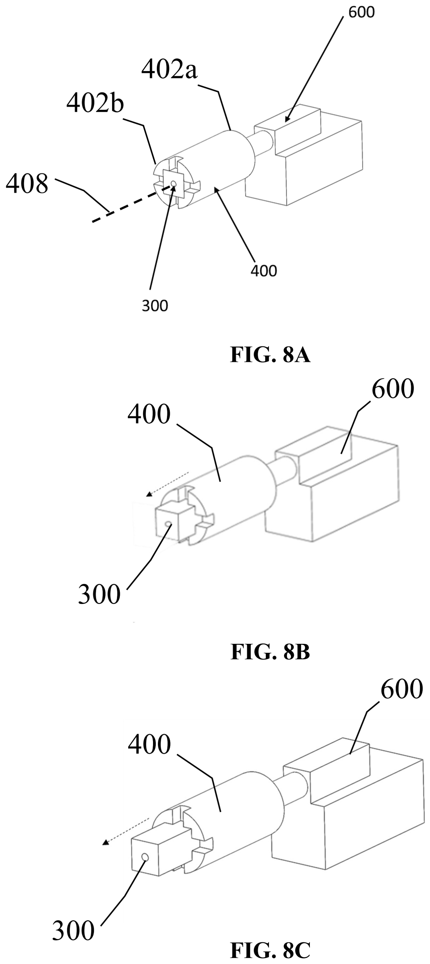

With reference to FIGS. 8A, 8B, and 8C, a drive assembly 600 is configured to drive the tissue sample 300, or any member configured to hold or support the tissue sample, in a specified direction. In the illustrated embodiment, the drive assembly 600 is located at the proximal end 402a of the tissue holder 400. In some embodiments, the drive assembly is configured to drive the tissue sample from the proximal to the distal end of the tissue holder along the axis 408 and out through the opening 407 at the distal end 402b. In some embodiments, the drive assembly 600 is configured to drive the tissue sample out of the tissue holder in incremental steps, thereby exposing at each step, a portion of the tissue sample through the opening at the distal end of the tissue holder. For example, FIG. 8C illustrates the tissue sample driven out at least one additional step from FIG. 8B.

In some embodiments, the drive assembly (or a component thereof) is configured to be coupled with the tissue sample via a member holding the tissue sample (e.g., the sacrificial tissue-support 412). In some embodiments, the tissue sample is supported on the sacrificial tissue-support and the drive assembly is coupled to the sacrificial tissue-support. In some embodiments, a portion of the drive assembly or a component thereof is configured to extend into the hollow cavity of the tissue holder. In some embodiments, the drive assembly comprises a linear actuator. Various kinds of linear actuation mechanism can be envisaged by a person skilled in the art. In some embodiments, the linear actuator comprises leadscrew, where the turning motion of the leadscrew is translated into a linear motion to drive the tissue sample. In some embodiments, if finer positional adjustments are required, the linear actuator comprises a piezoelectric actuator. In some embodiments, the linear actuator is configured to couple with the tissue sample via the sacrificial tissue-support. In some embodiments, the drive assembly comprises a gear mechanism, wherein a rack and pinion type of linear actuation can be envisaged. In some embodiments, a member holding the tissue sample (such as the sacrificial tissue-support) has edges designed on its sides. These edges interface with the circular gear mechanism (or the pinion) and the spinning gear action drives the tissue sample forward and optionally backward. In some embodiments, the drive assembly is configured to cause a two-way motion, that is to drive the tissue sample out of the hollow cavity of the tissue holder and retract the tissue sample back into the hollow cavity.

In some embodiments, the cutting system includes a rotating device 500 (such as shown in FIGS. 1A-3C) that is configured to cause relative rotational movement between the tissue holder 400 and the first cutting component 100 from a first relative orientation to a second relative orientation. In some embodiments, the rotating device 500 is coupled to the tissue holder and/or to the first cutting component. In some embodiments, the rotating device comprises a cam element. In some embodiments, the rotating device comprises a stepper motor or a servo motor coupled to the tissue holder. In some embodiments, the rotating device comprises a linear actuator, whose push/pull movement translates to a rotational movement.

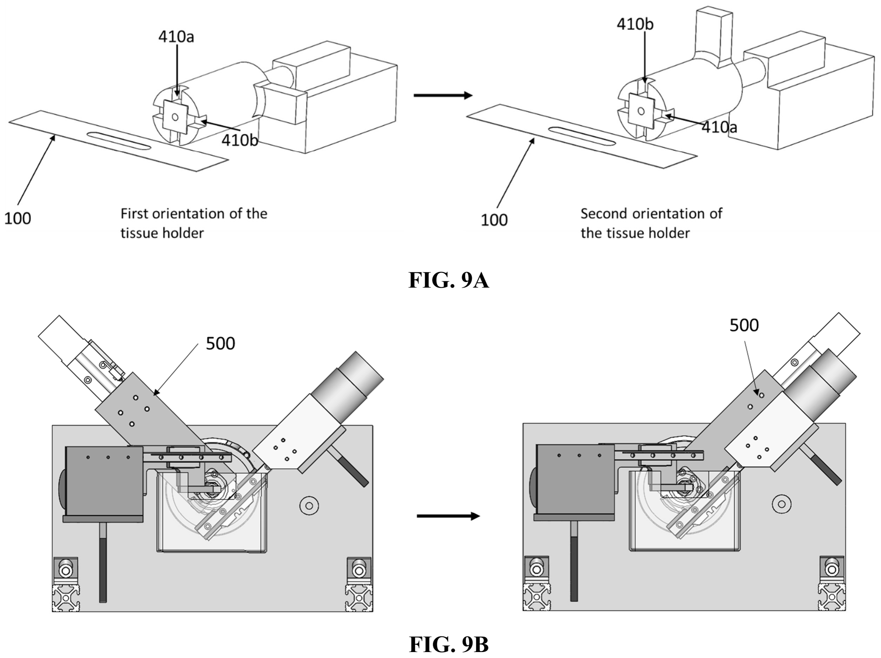

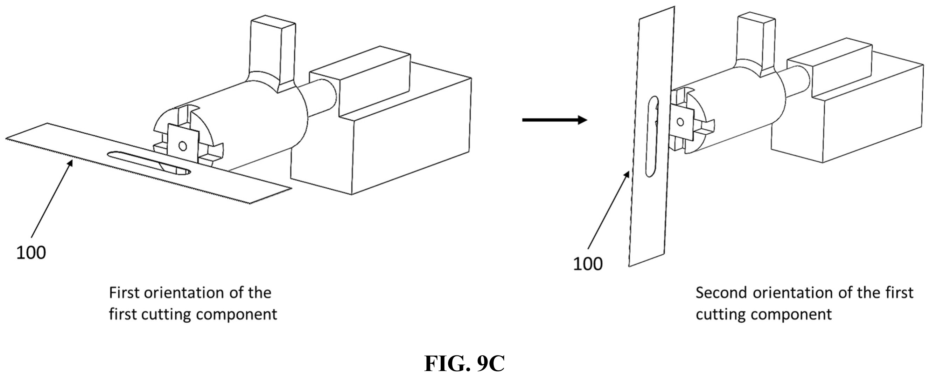

A relative orientation is the rotational orientation of the tissue holder 400 relative to the first cutting component 100. As used herein, "a first relative orientation" is an orientation of the tissue holder 400 relative to the first cutting component 100, wherein the first cutting component 100 is configured to cut the tissue sample 300 contained within the tissue holder at a cutting plane in a first dimension. As used herein, "a second relative orientation" is an orientation of the tissue holder 400 relative to the first cutting component 100, wherein the first cutting component 100 is configured to cut the tissue sample 300 contained within the tissue holder 400 at a cutting plane in a second dimension. A relative orientation can be achieved by rotating the tissue holder about the longitudinal axis 408 without rotating the first cutting component (such as shown in FIGS. 9A and 9B), or by rotating the first cutting component (such as about its transverse axis) without rotating the tissue holder (such as shown in FIG. 9C), or by rotating both.

In some embodiments, the cutting system includes a translation assembly 800 configured to cause a translational motion of, for example, a cutting component and/or the tissue holder. A translation assembly can comprise various types of translation stages with linear actuators to cause motion in one or more axes (such as a linear translation stage for single axis linear motion, an x-y translation stage for motion in x and y axes, or xyz translation stage for motion in x, y and z axes). Non-limiting examples of linear actuation mechanisms include an electro-mechanical actuator (for example, a screw-type actuator such as a leadscrew or a wheel and axel-type actuator such as a rack and pinion, and the like). In some embodiments, if finer positional adjustments are required, a translation assembly can comprise a piezoelectric actuator. In some embodiments, the tissue cutting system comprises one or more translation assemblies to drive translational motion of the tissue holder and/or a cutting component with respect to each other to cut the tissue sample supported by the tissue holder. In some embodiments, one or more translation assemblies cause translational motion of the tissue holder with respect to the cutting components. In some embodiments, one or more translation assemblies 800 (such as comprising an x-y translation stage shown in FIGS. 1A and 3A) drive the tissue holder towards and against the cutting components at a defined speed, such as depending upon the tissue type and presence of other materials such as encapsulants that surround the tissue. In some embodiments, the speed ranges between about 0.05 mm/sec and about 10 mm/sec.

With reference to FIGS. 1A, 1B, and 2A-2E, the cutting system includes a reservoir 1000 comprises an internal space surrounded by a surrounding wall and a horizontal base at the bottom. The internal space is configured to receive the tissue fragments. In some embodiments, the internal space is filled with a fluid material, such as a buffer, a saline solution or a culture medium (also called a cutting medium). In some embodiments, a portion of the tissue holder protrudes into the reservoir. In some embodiments, a portion of the tissue holder protrudes into the internal space of the reservoir, through an opening 1010 in the surrounding wall of the reservoir (see, for example, FIG. 2D). In some embodiments, the tissue holder or a portion thereof, is submerged within the fluid material filling the internal space of the reservoir. In some embodiments, the tissue holder and the reservoir are mounted on a translation stage (such a x-y translation stage). In some embodiments, the x-y translation stage causes an x-y translational motion of the tissue holder 400 and the reservoir 1000, with respect to the first cutting component 100 and/or the second cutting component 200. In some embodiments, the motion of the tissue holder containing the tissue sample towards and against the first cutting component and the second cutting component causes the cutting components to be driven into and penetrate the tissue sample, thereby cutting the tissue sample by scoring and slicing cuts respectively. In some embodiments, once the cut is made, the translation stage retracts the reservoir and the tissue holder back to the original position.

The internal space of the reservoir 1000 is temperature controlled. In some embodiments, the internal space of the reservoir 100 is configured to be heated. In some embodiments, the internal space of the reservoir 1000 is configured to be cooled (such as at temperatures close to 0 °C), without freezing the tissue or the tissue fragments. In other words, the reservoir 1000 is chilled without contaminating the contents of the internal space with ice or other chilling fluid.

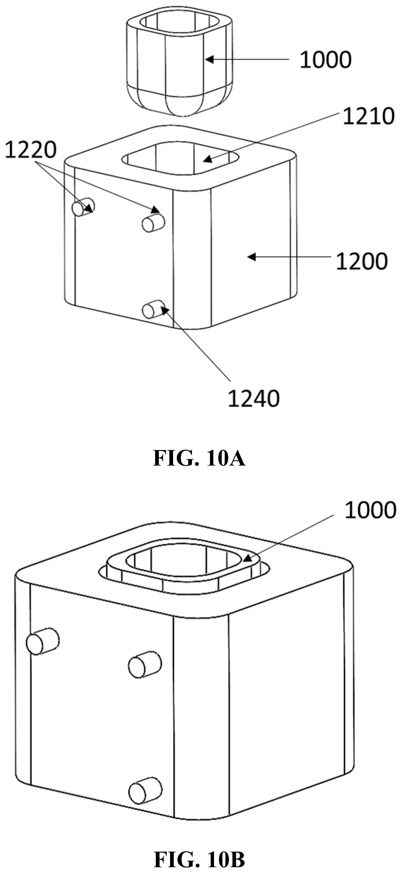

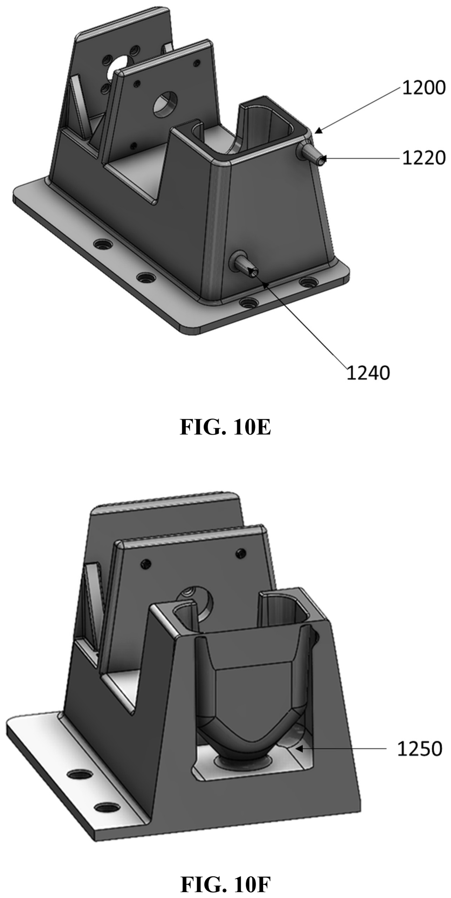

In some embodiments, an indirect temperature control mechanism is utilized. In some embodiments, as shown in FIG. 10A, this indirect temperature control mechanism is implemented with the help of a nest 1200 (e.g., a reservoir-nest), comprising an internal cavity 1210 matching the outer dimensions of the reservoir 1000. As shown in FIG. 10B, the nest 1200 is configured to hold the reservoir 1000 within the internal cavity 1210. In the illustrated embodiment, the nest 1200 surrounds the reservoir 1000 on all sides and the bottom, leaving only the top exposed. In some embodiments, a heat transfer fluid is configured to be circulated through the nest. The heat transfer fluid circulated through the reservoir-nest, maintains the temperature of the reservoir positioned within the nest, without coming in contact with the contents of the reservoir. Advantageously, this avoids any contamination of the tissue or the tissue fragments collected in the internal space.

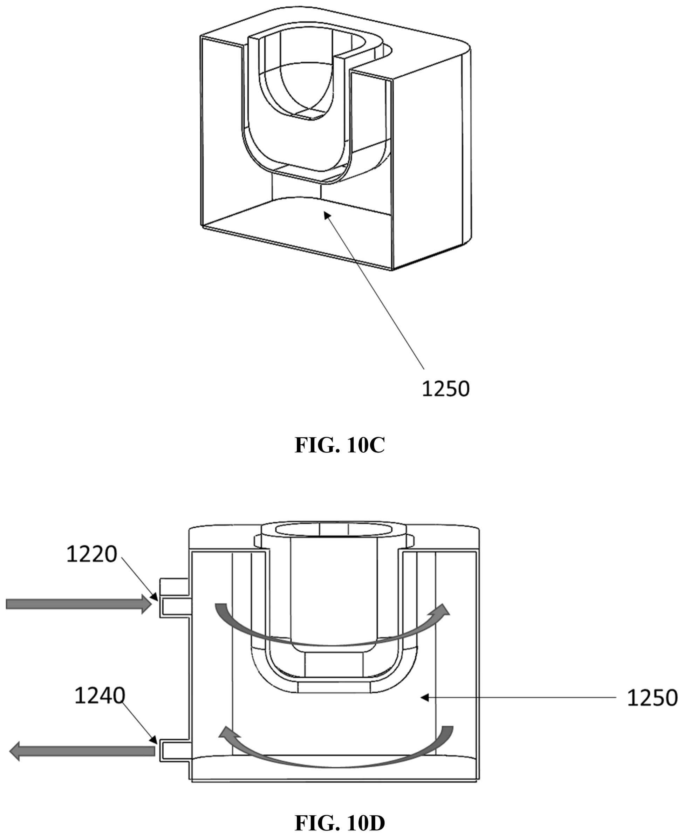

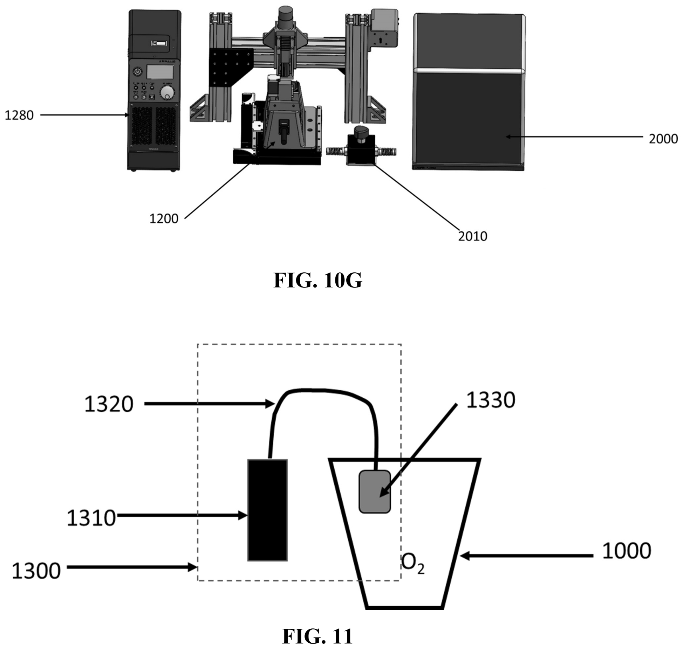

In some embodiments, the nest 1200 comprises internal features to enable circulation of the heat transfer fluid. In some embodiments (as shown by the section views in FIGS. 10C, 10D, and 10F), the internal feature is an enclosed space 1250 adjacent to the wall of the reservoir-nest. In some embodiments, as shown by the thick arrows in FIG. 10D, the heat transfer fluid circulates through the enclosed space. In some embodiments, the chilling fluid is supplied from an external heat exchanger 1280 (as shown in FIG. 10G). In some embodiments, the circulation of the heat transfer fluid is achieved with the help of insulated tubes that enable the flow of the heat transfer fluid between the external heat exchanger 1280 and the nest 1200. In some embodiments, the internal feature (e.g., the enclosed space adjacent to the wall of the nest) includes an inlet 1220 and an outlet 1240 for the inflow and the outflow of the heat transfer fluid. In some embodiments, the external heat exchanger is set at a temperature that translates to the final, desired temperature in the reservoir. For example, a temperature set to about -1 °C in the external heat exchanger, achieves a temperature of about 2.5 °C in the reservoir. In some embodiments, the external heat exchanger is turned on at the beginning of cutting protocol to ensure that the reservoir is chilled at desired temperature during the entire time of the cutting process. In some embodiments, this temperature is derived empirically and/or optimized with computational modeling for better performance predictability. The heat transfer fluid can be any standard coolant of suitable viscosity to enable smooth flow of the heat transfer fluid and thermal properties to maintain the desired temperature. Non-limiting examples of heat transfer fluid include liquids with freezing points lower than that of water. Examples of such liquids include ethylene glycol, propylene glycol and the like. In some embodiments, the internal space of the reservoir is configured to be maintained at a temperature between about 1 °C and about 10 °C (such 1 °C, 1.5 °C, 2 °C, 2.5 °C, 4 °C, 5 °C and so on). While the temperature is held reasonably steady, there can be variations within ± 0.5 °C. In some embodiments, the internal space of the reservoir is configured to be maintained at a temperature between about 1 °C and about 4 °C.

With reference to FIG. 1B and 11, the cutting system includes an oxygenation unit 1300 configured to supply oxygen (O₂) to the internal space of the reservoir 1000 during the cutting processes. In some embodiments, the oxygenation unit 1300 is coupled to the reservoir 1000. The oxygenation unit 1300, in non-limiting embodiments, can comprise an O₂ source 1310 such as an oxygen tank, an oxygen generator, an oxygen concentrator, or a cannister, wherein the O₂ source is external to the reservoir. In some embodiments, oxygenation of the cutting medium (that is the fluid material filling the internal space of the reservoir) during the cutting process was found to preserve the viability of the tissue fragments. In some embodiments, the oxygenation unit is coupled to the reservoir with the help of an interface 1330 that cause minimum disturbance of the tissue fragments collected in the reservoir. In some embodiments, the oxygenation unit comprises an oxygen source 1310 (such as O₂ tank, generator, concentrator and the like), a pressure regulator (not shown) to control the O₂ pressure at the outlet of the O₂ source, and a tube 1320 extending from the O₂ source into the internal space of the reservoir. In some embodiments, the oxygenation unit interfaces with air stones in the cutting medium contained within the internal space of the reservoir.

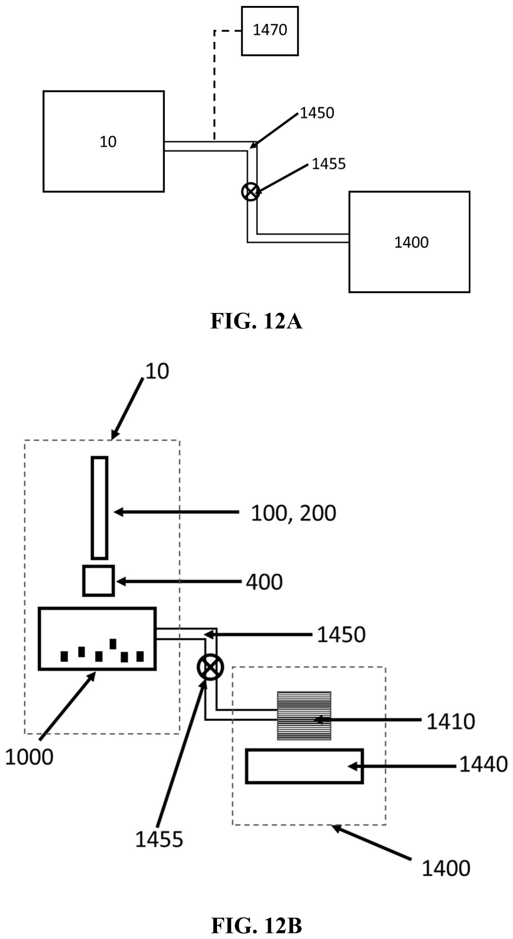

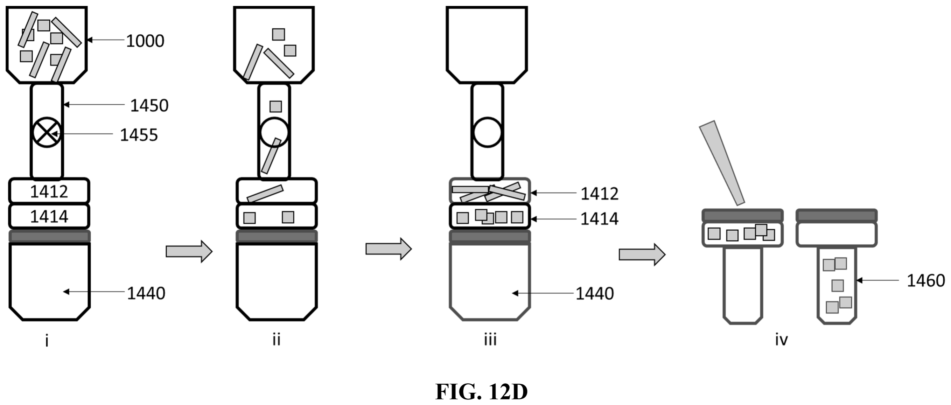

With reference to FIGS. 12A-12D, a cutting system 10 includes a filtration system 1400 comprises a filter assembly 1410. The filter assembly 1410 filters out tissue debris, tissue fragments not of the specified (desired) size and/or other unwanted substances (such as fragments of sacrificial tissue-support, adhesive material, encapsulants etc.) in order to enrich for tissue fragments of a specified size. The filter assembly 1410 comprises at least one filter unit. In some embodiments, the filter assembly comprises a plurality of filter units, of different pore sizes. In some embodiments, the plurality of filter units are connected in series (FIG. 12D), wherein the filtrate from a first filter unit passes through a second filter unit and so on. In some embodiments, the filter assembly comprises one or more integrated filter units. In some embodiments, the filter assembly with one or more integrated filter units is washable and reusable. In some embodiments, the filter assembly allows the attachment of one or more off-the-shelf or customized filter units as required by the user.

With reference to FIG. 12D, in some embodiments, the filter assembly comprises a first filter unit 1412 of a first (e.g., a larger) pore size to retain unwanted tissue fragments of size bigger than a specified size and allow the desired tissue fragments (such as of the specified size) along with other smaller debris to flow through. In some embodiments, the surface area of this first filter unit is large enough to prevent the clogging of the pores by the larger fragments and other large particles, which would block the passage of the smaller (and the desired) tissue fragments. In some embodiments, the filter assembly further comprises a second filter unit 1414 of a second (smaller) pore size to retain tissue fragments of the specified size and allow the smaller debris to flow through. In some embodiments, the filtration system further comprises a flushing mechanism configured to supply a wash buffer at a desired flow rate for washing the filter units in order to dislodge any particles that clog the pores. In some embodiments, the filtration system further comprises mechanical agitators to dislodge particles that block the pores. In some embodiments, the filtration system comprises a first collection tank 1440 to collect the filtrate (such as comprising debris and unwanted materials). In some embodiments, the filtration system comprises a fragment collection tank 1460 to collect the tissue fragments of specified size.

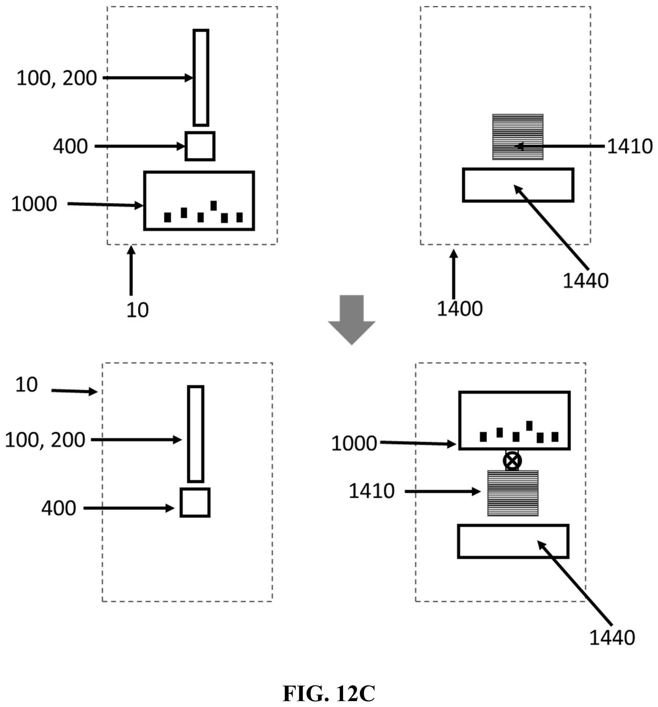

In some embodiments, the filter assembly 1410 is configured to be coupled to the reservoir 1000. In some embodiments, the filter assembly is coupled to the reservoir at the end of the cutting operation once the entire tissue has been cut into tissue fragments. In some embodiments, the filtration system is an in-line filtration system (FIG. 12B). The filtration process occurs at the original location of the reservoir within the tissue cutting system, wherein the filter assembly is coupled to the reservoir at the original location of the reservoir within the tissue cutting system. In other embodiments, the filtration system is an off-line filtration system (FIG. 12C). In some embodiments, after completion of the cutting operation, the reservoir with the desired tissue fragments, along with other undesired fragments and debris, is transferred from the cutting region (within the tissue cutting system 10) to the filtration system 1400, comprising the filter assembly 1410. The transfer can be a manual or an automated transfer. In some embodiments, the filtration system 1400 is configured to be coupled to the tissue cutting system for an automated transfer of the reservoir from a first location within the tissue cutting system to a second location within the filtration system. In some embodiments, the reservoir is inverted on the filter assembly.

In some embodiments, the reservoir is connected to the filter assembly with the help of a connector 1450 (such as shown in FIG. 12A and 12D(i). In some embodiments, the connector 1450 comprises a valve 1455. During the filtration process, the valve 1455 is opened as shown in FIG. 12D(ii) to allow the contents of the reservoir 1000 to flow into the filter assembly 1410. In some embodiments, as shown in FIG. 12D(iii), undesired bigger fragments are retained on the first filter unit 1412, while tissue fragments (of the specified size) flow through. In some embodiments, tissue fragments (of specified size) are retained on the second filter unit 1414 (such as shown in FIG. 12d (iii)), while smaller debris flow through into the collection tank 1440. In some embodiments, the first and second filter units are connected in series in the filter assembly. In some embodiments, the second filter unit 1414 is disassembled from the filter assembly after the filtration process and coupled to a fragment collection tank 1460 to collect the tissue fragments of the specified size, such as shown in FIG. 12D(iv). In some embodiments, the contents of the reservoir (such as tissue fragments of specified size along with other unwanted materials, such as bigger fragments, debris and other impurities) flow into the filtration system by a passive flow driven by gravity. In some embodiments, the filtration system further comprises a fluidic drive mechanism 1470 (FIG. 12A) coupled to the connector 1450 and configured to drive the contents of the reservoir into the filter assembly. Suitable fluidic drive mechanism includes pumps, positive air pressure systems and the like.

As detailed herein, some embodiments relate to a tissue cutting system comprising, a tissue holder 400 comprising a hollow cavity defined by a sidewall, a proximal end and an opening at a distal end, wherein the tissue holder is configured to support a tissue sample 300 within the hollow cavity. The tissue cutting system further comprises a first cutting component 100 configured to cut a tissue sample contained within the hollow cavity by scoring cuts in a first dimension and in a second dimension respectively, to produce a scored tissue sample. In some embodiments, the tissue cutting system further comprises a rotating device 500 configured to cause a relative rotational movement between the tissue holder and the first cutting component, from a first relative orientation to a second relative orientation. In the first relative orientation, the first cutting component is configured to cut the tissue sample (contained within the hollow cavity of the tissue holder) in the first dimension, and in the second relative orientation, the first cutting component is configured to cut the tissue sample (contained within the hollow cavity of the tissue holder) in the second dimension. The tissue cutting system further comprises a second cutting component configured to cut the scored tissue sample in a third dimension to produce tissue fragments.

In some embodiments, the tissue holder 400 further comprises a sacrificial tissue-support 412, wherein the sacrificial tissue-support is configured to support the tissue sample. In some embodiments, the sacrificial tissue-support comprises a non-flat surface comprising a groove, wherein the groove is configured to support the tissue sample. In some embodiments, the second cutting component is configured to cut the tissue sample by cutting through the sacrificial tissue-support. In some embodiments, the non-flat surface is a V-shaped surface.

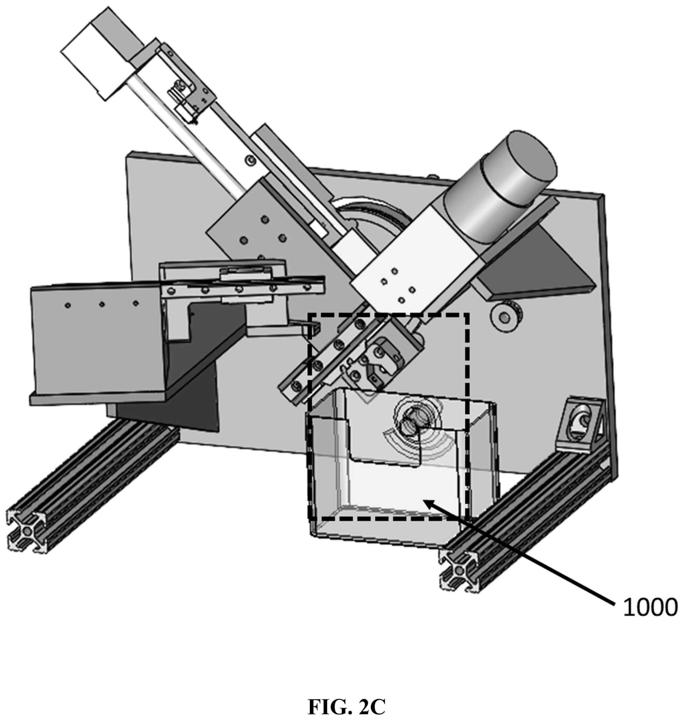

In some embodiments, the rotating device 500 is coupled to the tissue holder and is configured to rotate the tissue holder about its longitudinal axis from a first relative orientation of the tissue holder to a second relative orientation of the tissue holder. In some embodiments, the rotating device 500 is coupled to the tissue holder 400 via a coupling element 502 (such as shown in FIG. 2C and 2D). In some embodiments, the first cutting component is not rotated or is rotationally fixed. In some embodiments where the tissue holder is rotated and not the first cutting component (such as shown in FIG. 9A and 9B), the first relative orientation is referred to as the "first orientation of the tissue holder" and the second relative orientation is referred to as the "second orientation of the tissue holder". In the first orientation of the tissue holder, the first cutting component is configured to cut a tissue sample (contained within the tissue holder) at a cutting plane in the first dimension and in the second orientation of the tissue holder, the first cutting component is configured to cut the tissue sample (contained within the tissue holder) at a cutting plane in the second dimension. In some embodiments, in the first orientation of the tissue holder, the first cutting component is configured to cut the tissue sample at successive cutting planes in the first dimension and in the second orientation of the tissue holder, the first cutting component is configured to cut the tissue sample at successive cutting planes in the second dimension. In some embodiments, the rotating device is configured to rotate the tissue holder by an angle of 90° about its longitudinal axis.

In some embodiments, the rotating device 500 is coupled to the first cutting component and is configured to rotate the first cutting component (such as about its transverse axis) from a first orientation of the first cutting component to a second orientation of the first cutting component (such as shown in FIG. 9C). In embodiments where the first cutting component is rotated and not the tissue holder, the first relative orientation can be referred to as the "first orientation of the first cutting component" and the second relative orientation can be referred to as the "second orientation of the first cutting component". In the first orientation of the first cutting component, the first cutting component is configured to cut the tissue sample (contained within the tissue holder) at a cutting plane in the first dimension, and in the second orientation of the first cutting component, the first cutting component is configured to cut the tissue sample (contained within the tissue holder) at a cutting plane in the second dimension. In some embodiments, the rotating device is configured to rotate the first cutting component by an angle of 90°.

In some embodiments, the tissue holder 400 or the first cutting component 100 is configured to be moved (e.g., vertically or side-ways) with respect to each other. In some embodiments, a translation assembly is configured to cause the translational motion of the tissue holder and/or the first cutting component. In some embodiments, the tissue cutting system comprises a translation assembly coupled to the tissue holder and configured to cause a translational motion of the tissue holder with respect to the first cutting component. In some embodiments, the translation assembly is configured to move the tissue holder between successive translational positions (such as from a n-1^{th} translational position to a n^{th} translational position) with respect to the first cutting component. In some embodiments, the successive translational positions are vertical translational positions. In some embodiments, the successive translational positions are side-ways (or horizontal) translational positions. In some embodiments, at each translational position (such as a vertical or a horizontal translational position) of the tissue holder, the first cutting component is configured to be moved towards and away from the tissue sample contained within the tissue holder, thereby cutting the tissue sample at successive cutting planes. In some embodiments, at each translational position (such as a vertical or a horizontal translational position) of the tissue holder, the tissue holder containing the tissue sample is configured to be moved towards and away from the first cutting component, thereby causing the tissue sample to be cut at successive cutting planes by the first cutting component. In some embodiments, translational movement of the tissue holder is a vertical translational movement, wherein n-1^{th} translational position is above or below the n^{th} translational position. In some embodiments, the translational movement of the tissue holder is a side-ways (such as horizontal translational movement), wherein the n-1^{th} translational position is on the side (such as left or right) of the n^{th} translational position.

In some embodiments, the tissue cutting system comprises a vertical translation assembly configured to cause a vertical movement of the tissue holder and the first cutting component with respect to each other. In some embodiments, a vertical translation assembly is configured to cause a vertical movement of the tissue holder with respect to the first cutting component. In some embodiments, the tissue cutting system comprises a vertical translation assembly coupled to the tissue holder and configured to move the tissue holder containing the tissue sample to successive vertical translational positions with respect to the first cutting component. The vertical movement can be upward and/or downward movement. In some embodiments, the vertical translation assembly moves the tissue holder from a n-1^{th} vertical position to an nth vertical position (such as from a 1^{st} vertical position to a 2^{nd} vertical position, from a 2^{nd} vertical position to a 3^{rd} vertical position and so on). The n^{th} vertical position can be below or above the n-1^{th} vertical position depending on whether the tissue holder is moved downwards of upwards. "Moved vertically" or "vertical movement" used in the context of the tissue holder (or the first cutting component) is not necessarily a movement in a direction perpendicular to a horizontal plane. A vertical movement is a movement that moves the tissue holder in an upward or a downward direction with respect to the first cutting component (or vice versa) from a n-1^{th} vertical position to a n^{th} vertical position, wherein the n-1^{th} vertical position is below or above the n^{th} vertical position. The vertical positions need not necessarily be directly one above the other. In other words, side-ways vertical movement and staggered vertical positions are also within the scope of this invention. In some embodiments, the movement of the tissue holder between successive vertical positions can be precisely controlled, such as with a piezoelectric actuator. In some embodiments, the translation assembly (such as the vertical translation assembly) is configured to position the tissue sample (such as by vertically moving the tissue holder) with respect to the first cutting component, wherein the first cutting component cuts the tissue sample at a desired cutting plane. In some embodiments, the translation assembly moves the tissue holder from a 1^{st} vertical translational position (where a first cutting event occurs) to a 2^{nd} vertical translational position (where a second cutting event occurs) and so on.

In some embodiments, the tissue cutting system comprises a translation assembly configured to cause a horizontal movement of the tissue holder and the first cutting component with respect to each other. In some embodiments, the translation assembly comprises a x-y translation stage. In some embodiments, the tissue holder is mounted on the x-y translation stage. In some embodiments, the x-y translation stage is configured to move the tissue holder (containing the tissue sample) to successive horizontal positions (wherein the n-1^{th} translational position is on the side, such as on the left or on the right of the n^{th} translational position) with respect to the first cutting component. In some embodiments, at each horizontal translational position of the tissue holder, the x-y translation stage is further configured to move the tissue holder towards and away from the first cutting component, thereby enabling the first cutting component to make successive scoring cuts in the tissue sample contained within the tissue holder.

In some embodiments, the first cutting component is located near the opening at the distal end of the tissue holder. In some embodiments, the first cutting component is configured to be moved towards and away from the tissue sample or the tissue holder containing the tissue sample is configured to be moved towards and away from the first cutting component (scoring motion), to cut the tissue sample by a scoring cut at a cutting plane. When the first cutting component moves towards the tissue sample or the tissue sample (contained within the tissue holder) is moved towards the first cutting component, it is to be understood that the first cutting component moves into (such as to penetrate) the tissue sample, thereby cutting the tissue sample at a cutting plane. After the tissue sample is cut at the cutting plane, the first cutting component or the tissue holder is retracted to the original position. In some embodiments, at each vertical or horizontal position of the tissue holder, the first cutting component is configured to be moved towards and away from the tissue sample contained within the tissue holder, to cut the tissue sample at successive cutting planes. In some embodiments, at each vertical or horizontal position of the tissue holder, the tissue holder containing the tissue sample is configured to be moved towards and away from the first cutting component to cut the tissue sample at successive cutting planes.

In some embodiments, the first cutting component is configured to be oscillated during the first and the second cutting processes. In some embodiments, the first cutting component is configured to be oscillated at a frequency of about 20 Hz to about 200 Hz. In some embodiments, the first cutting component is configured to be oscillated at frequencies within a range of approximately 50 to approximately 200 Hz. In some embodiments, the first cutting component is configured to be oscillated at frequencies within a range of approximately 120 to approximately 200 Hz. In some embodiments, the oscillation of the first cutting component about its axes and the movement of the first cutting component towards and away from the tissue sample (scoring motion) are caused by a voice coil actuator. In some embodiments, the oscillation motion and the scoring motion of the first cutting component are synchronous.

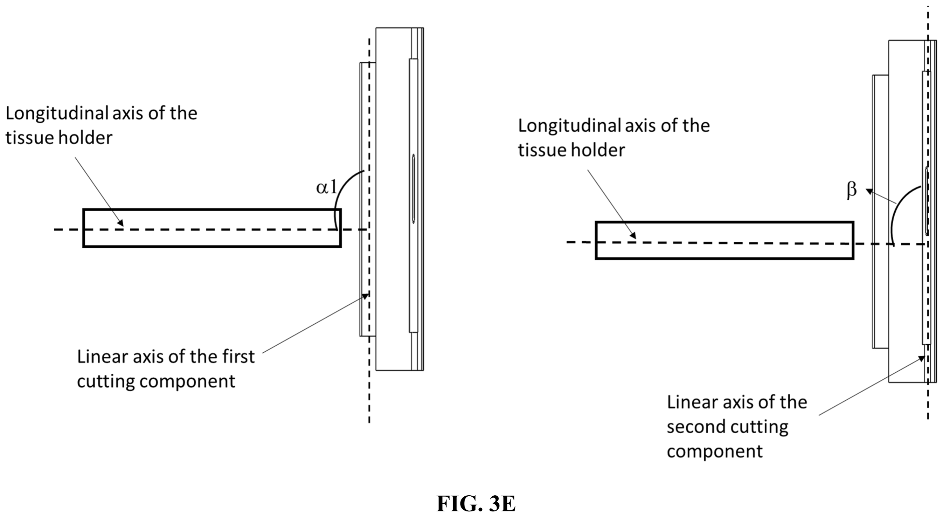

In some embodiments, the first cutting component is oriented with respect to the tissue holder (such as during the first and the second cutting processes), wherein the linear axis of the first cutting component is at an angle α1 with respect to the tissue holder (such as the longitudinal axis of the tissue holder). In some embodiments, where the tissue holder is oriented horizontally (such as where the longitudinal axis of the tissue holder is parallel to a horizontal plane), α1 is the angle that the linear axis of the first component makes with respect the horizontal plane. In some embodiments, α1 is about 20°. In some embodiments, as shown in representative FIG. 3E, α1 is about 90°. In some embodiments, during the cutting events in the first and the second dimensions, the first cutting component is configured to approach the tissue sample (enabled by a translational motion of the first component and/or the tissue holder) in an orientation, wherein the plane of the first cutting component makes an angle α2 (angle of approach as shown in FIG. 4E) to the surface of the tissue sample being cut. In some embodiments, α2 is 90°. In some embodiments, α2 is an oblique angle (wherein α2 < 90°).

In some embodiments, the first cutting component is configured to cut the tissue sample while the tissue sample is contained within the hollow cavity of the tissue holder. This prevents any wobble of the tissue sample during the first and the second cutting processes, thereby enabling the first cutting component to make precise scoring cuts. In some embodiments, the optimal design and dimensions of the tissue holder ensure that there is space to allow the first cutting component to cut the tissue sample, while the tissue sample is held stably.

In some embodiments, the sidewall of the tissue holder comprises a slit, wherein the slit permits the first cutting component to pass through and cut the tissue sample while the tissue sample is contained within the hollow cavity of the tissue holder. In some embodiments, the sidewall of the tissue holder comprises a plurality of slits, wherein each slit in the plurality of slits permits the first cutting component to pass through and cut the tissue sample while the tissue sample is contained within the hollow cavity of the tissue holder. In some embodiments, the width of each slit 410(w) is between about 1 mm and about 10 mm. In some embodiments, the spacing 410(s) between two adjacent slits in a plurality of slits is between about 300 µm and about 2000 µm. In some embodiments, the depth 410(d) of the slit is between about 50 µm and about 1000 µm.

In some embodiments, the first cutting component is configured to protrude within the hollow cavity through the opening at the distal end and cut the tissue sample while the tissue sample is contained within the hollow cavity of the tissue holder.

In some embodiments, the tissue cutting system further comprises a reservoir 1000 (such as shown in FIGS. 1A, 1B, and 2A-2E). In some embodiments, the reservoir comprises an internal space surrounded by a surrounding wall and a horizontal base. The internal space is configured to receive the tissue fragments. In some embodiments, the internal space is filled with a fluid material (used interchangeably with a cutting medium), such as a buffer, a saline solution or a culture medium. In some embodiments, the internal space of the reservoir is configured to be maintained at a temperature between about 1 °C and about 10 °C. In some embodiments, the internal space of the reservoir is configured to be maintained at a temperature of about 1 °C and about 4 °C. In other embodiments, the internal space of the reservoir is maintained at a temperature within a range of approximately 35°C to approximately 38 °C . In some embodiments, a portion of the tissue holder protrudes into the reservoir. In some embodiments, a portion of the tissue holder protrudes into the internal space of the reservoir, through an opening 1010 in the surrounding wall of the reservoir (see for example FIG. 2D). In some embodiments, the tissue holder or a portion thereof, is submerged within the fluid material filling the internal space of the reservoir.

In some embodiments, as shown in FIGS. 10A-10F, the tissue cutting system comprises a nest 1200 configured to hold the reservoir within an internal cavity of the nest, wherein the nest comprises an internal feature 1250 for circulating a heat exchange fluid through the nest and wherein the heat exchange fluid circulated through the nest maintains the temperature of the reservoir between about 1 °C and about 10 °C. In some embodiments, the nest is connected to an external heat exchanger 1280 (FIG. 10G) which supplies the heat exchange fluid to the nest and wherein the desired temperature of the reservoir is achieved by pre-setting the temperature of the external heat exchanger.

In some embodiments, as shown in FIG. 11, the tissue cutting system comprises an oxygenation unit 1300 comprising an oxygen source 1310, wherein the oxygen source is coupled to the reservoir and is configured to oxygenate the fluid material in the internal space of the reservoir.

In some embodiments, as shown in FIGS. 12B-12D, the reservoir is configured to be coupled to a filter assembly 1410, wherein the filter assembly comprises at least a first filter unit 1412 of a first (bigger pore size) to retain undesired tissue fragments of size larger than a specified size and allow the passage of desired tissue fragments of the specified size. In some embodiments, the filter assembly further comprises at least a second filter unit 1414 of a second (smaller pore size) connected in series with the first filter unit to retain the desired tissue fragments of the specified size. For example, if the specified size is 300 µm × 300 µm × 300 µm, the first filter unit will have a pore size that is big enough to allow the tissue fragments of the specified size (with permitted size variation) to flow through, while retaining tissue fragments greater than the specified size. The second filter unit will have a pore size to retain the tissue fragments of the specified size, while allowing tissue debris and unwanted material of smaller dimension to flow through.

In some embodiments, the tissue holder is configured to be driven towards the second cutting component. In some embodiments, the tissue holder and the reservoir are configured to be driven towards the second cutting component. In some embodiments, the translation assembly (such as comprising the x-y translation stage) is configured to drive the tissue holder and the reservoir towards the second cutting component. In some embodiments, the translation assembly is coupled to the tissue holder. In some embodiments, the translation assembly comprises a linear actuator mechanism that causes a bulk directional motion, in a forward as well as a reverse direction. In some embodiments, the translation assembly 800 comprises a translation stage (such as a x-y translation stage). In some embodiments, such as shown in FIG. 1A, the tissue cutting system comprises a translation stage on which the reservoir and the tissue holder are mounted, wherein the translation stage is configured to cause a translation motion of the reservoir and the tissue holder towards and away from the first cutting component and/or the second cutting component. The movement of the tissue holder towards the cutting components (the first and/or the second) causes the tissue sample contained within the tissue holder to be driven against the cutting components, thereby causing the cutting components to penetrate the tissue sample and cut the tissue sample at a cutting plane. In some embodiments, the tissue holder moves against the first cutting component by a specified distance, wherein the first cutting component penetrates the tissue sample up to a shallow depth, thereby cutting the tissue sample by scoring cuts. In some embodiments, the first cutting component cuts the tissue sample while the tissue sample is contained within the tissue holder. In some embodiments, the first cutting component cuts the tissue sample by passing through the slits in the sidewall of the tissue holder.

In some embodiments, the tissue cutting system further comprises a drive assembly 600 configured to be coupled to the tissue sample, wherein the drive assembly is configured to drive out a portion of the scored tissue sample through the opening at the distal end to expose a portion of the scored tissue sample. In some embodiments, the drive assembly is configured to be coupled to the tissue sample via a sacrificial tissue-support. Any portion of the tissue sample that is exposed out of the hollow cavity of the tissue holder, such as through the opening at the distal end of the tissue holder, is referred to as an exposed portion of the tissue sample. Any portion of a scored tissue sample that is exposed out of the hollow cavity of the tissue holder, such as through the opening at the distal end of the tissue holder, is referred to as an exposed portion of the scored tissue sample. In some embodiments, the second cutting component is configured to cut an exposed portion of the scored tissue sample. In some embodiments, the exposed portion of the scored tissue sample is configured to be driven across the second cutting component. In some embodiments, the drive assembly and/or the translation assembly is configured to drive the exposed portion of the scored tissue sample across the second cutting component.

In some embodiments, the second cutting component is located at the distal end of the tissue holder, that is, near the opening at the distal end of the tissue holder. In some embodiments, the second cutting component is located beyond the first cutting component, that is, the second cutting component is located farther from opening at the distal end of the tissue holder than the first cutting component. In some embodiments, the second cutting component spans over the portion of the tissue holder that protrudes into the internal space of the reservoir. In some embodiments, the first and the second cutting components are mounted on a single cutting component holder but oriented at different angles with respect to the tissue sample.

In some embodiments, the second cutting component is configured to be oscillated. In some embodiments, the second cutting component is translationally fixed, and the only motion of the second cutting component is an oscillating motion about the linear axis of the second cutting component. In some embodiments, the second cutting component (such as the linear axis of the second cutting component) is oriented at an angle β with respect to the tissue holder (such as the longitudinal axis of the tissue holder). In some embodiments, the tissue holder is oriented parallel to the horizontal base of the reservoir and the second cutting component is oriented at an angle, wherein the linear axis of the second cutting component is at an angle β with respect to the horizontal base of the reservoir. In some embodiments, β is about 0° (wherein the linear axis of the second cutting component is parallel to the horizontal base of the reservoir). In some embodiments, β is between about 0° and about 40°. In some embodiments, such as shown in FIG. 3E, β is about 90° (wherein the linear axis of the second cutting component is perpendicular to the horizontal base of the reservoir). In some embodiments, the plane of the second cutting component is at an angle γ with respect to the surface of the tissue sample to be cut. In some embodiments, γ is between about 0.25° and about 30°. In some embodiments, γ is between about 0.5° and about 2.5°.

In some embodiments, as shown in FIG. 3A-3C, the tissue cutting system comprises a single oscillator (comprising a voice coil actuator), which is configured to oscillate both the first cutting component and the second cutting component at frequencies between about 20 Hz and about 200 Hz. In some embodiments, the oscillator 32 is connected to a linear rail system 34 comprising a cutting component holder 31, wherein the first cutting component and the second cutting component are mounted on the cutting component holder 31. In some embodiments, the planes of the first and the second cutting components are oriented at different angles with respect to the surface of the tissue sample contained within the tissue holder. In some embodiments, plane of the first cutting component is oriented an angle α2 with the surface of the tissue sample, while the plane of the second cutting component is oriented at an angle γ with the surface of the tissue sample. In some embodiments, α2 and γ are not equal. In some embodiments, α2 is about 90°. In some embodiments, γ is between about 0.5° and about 2.5°. As represented in FIG. 4E, angles α2 and γ are the angles of approach that the planes of the first and the second cutting components respectively make with the surface of the tissue sample. In some embodiments, as shown in FIG. 3D, while mounted on the holder, the plane of the first cutting component is at an angle δ with respect to the plane of the second cutting component. In some embodiments, such as shown in FIG. 3E, the first and the second cutting component are mounted on the single cutting component holder in an orientation, wherein α1 and β are both about 90°.

Some embodiments relate to a tissue cutting system for cutting a tissue sample comprising: a tissue holder comprising a hollow cavity defined by a side-wall, a proximal end, an opening at a distal end, and a sacrificial tissue-support within the hollow cavity of the tissue holder; wherein the sacrificial tissue-support is configured to support a tissue sample, and wherein the sacrificial tissue-support supporting the tissue sample is configured to be driven out of the hollow cavity to expose a portion of the tissue sample through the opening at the distal end. The tissue cutting system further comprises at least one cutting component configured to cut the tissue sample to produce tissue fragments, wherein the cutting component is configured to cut an exposed portion of the tissue sample, and wherein the cutting component is configured to cut the exposed portion of the tissue sample by cutting through the sacrificial tissue-support. In some embodiments, the tissue cutting system further comprises a reservoir filled with a fluid material, wherein a portion of the tissue holder supporting the tissue sample is configured to be submerged within the fluid material. In some embodiments, the exposed portion of the tissue sample is supported on the sacrificial tissue-support. In some embodiments, the tissue sample is a biopsy tissue sample, that is a tissue sample obtained by biopsy, wherein cutting in a single dimension is sufficient to produce tissue fragments of specified size. In some embodiments, the sacrificial tissue-support comprises a non-flat surface comprising a groove, wherein the groove is configured to support a tissue sample. In some embodiments, the sacrificial tissue-support is configured to support a plurality of tissue samples for simultaneous cutting of the plurality of tissue samples. In some embodiments, the sacrificial tissue-support comprises a non-flat surface comprising a plurality of grooves. In some embodiments, each groove in the plurality of grooves is configured to support a tissue sample for simultaneous cutting of a plurality of tissue samples.

In some embodiments, the reservoir is configured to receive the tissue fragments. In some embodiments, the surrounding wall of the reservoir comprises an opening 1010 to receive the tissue holder, wherein the tissue holder protrudes into the internal space of the reservoir through the opening. In some embodiments, the opening is fitted with an O-ring to prevent any leakage of the fluid material. In some embodiments, the fluid material is maintained at a temperature between about 1 °C and about 10 °C. In some embodiments, the fluid material is maintained at a temperature between about 35 °C and about 38 °C.

In some embodiments, the cutting component is located near the distal end of the tissue holder. In some embodiments, the cutting component spans over the tissue holder. In some embodiments, the cutting component spans over the portion of the tissue holder that protrudes into the internal space of the reservoir and is submerged within the fluid material. In some embodiments, the tissue holder is oriented parallel to a horizontal base of the reservoir and the cutting component is oriented at an angle, wherein the linear axis of the cutting component is at an angle (e.g., β) with respect to the horizontal base of the reservoir, wherein the angle is between about 0° and about 40°. In some embodiments, the plane of the cutting component is at an angle (e.g., γ) with respect to the surface of the tissue sample to be cut, wherein the angle is between about 0.25° and about 30°. In some embodiments, the cutting component is configured to be oscillated at a frequency between about 20 Hz and about 200 Hz. In some embodiments, the cutting component is translationally fixed. In some embodiments, the tissue cutting system further comprises a drive assembly, wherein the drive assembly is configured to drive out the sacrificial tissue-support to expose a portion of the tissue sample (supported on the sacrificial tissue-support) out of the hollow cavity of the tissue holder through the opening at the distal end. In some embodiments, the tissue cutting system further comprises a translation assembly. In some embodiments, the translation assembly comprises a translation stage on which the tissue holder is mounted. In some embodiments, the translation assembly and/or the drive assembly is configured to drive the exposed portion of the live tissue sample across the cutting component to cut the exposed portion of the live tissue sample into tissue fragments.

As shown in FIG. 3A-3C, some embodiments relate to a tissue cutting system comprising: a tissue holder configured to hold a tissue sample and a cutting assembly comprising: i) a linear rail system 34 comprising a cutting component holder 31; ii) a first cutting component and a second cutting component mounted on the cutting component holder; and iii) an oscillator 32 connected to the linear rail system and configured to oscillate the first cutting component and the second cutting component. In some embodiments, the first cutting component is configured to cut the tissue sample in a first dimension and a second dimension to produce a scored tissue sample, and the second cutting component is configured to cut the scored tissue sample in a third dimension to produce tissue fragments. In some embodiments, the tissue cutting system further comprises, a reservoir configured to hold the tissue fragments, wherein a portion of the tissue holder protrudes into an internal space of the reservoir. In some embodiments, the tissue holder is mounted on a translation stage. The translation stage is configured to cause a translational motion of the tissue holder containing the tissue sample towards and away from the first cutting component and the second cutting component. In some embodiments, the translation stage is further configured to cause successive side-ways (or horizontal) translational movement of the tissue holder with respect to the first cutting component. In some embodiments, the motion of the tissue holder containing the tissue sample towards the cutting components causes the cutting components to penetrate into the tissue sample, thereby causing the tissue sample to be cut at a cutting plane.

In some embodiments, the first cutting component and the second cutting are mounted on the cutting component holder in a relative orientation with respect to each other and with respect to the tissue holder, wherein the plane of the first cutting component is at an angle α2 (e.g., α2 is about 90°) with respect to the surface of a tissue sample contained within the tissue holder and wherein the plane of the second cutting component is at an angle γ (e.g., γ is between about 0.5° and about 2.5°) with respect to the surface of the tissue sample contained within the tissue holder. In some embodiments, as shown in FIG. 3D, while mounted on the cutting component holder, the plane of the first cutting component is at an angle δ with respect to the plane of the second cutting component. In preferred embodiments, the relative orientation of the cutting components (mounted on the holder) with respect to each other and with respect to the surface of the tissue sample contained within the tissue holder does not change during the entire cutting operation. In some embodiments, such as shown in FIG. 3e, the first and the second cutting component are mounted on the single cutting component holder in an orientation, wherein α1 and β are both about 90°. In some embodiments, the translation stage is configured to cause a translational motion of the tissue holder containing the tissue sample towards the first and the second cutting components at a specified speed. In some embodiments, the specified speed depends upon various factors such as the frequency and amplitude of oscillation of the cutting components, the tissue type, encapsulant surrounding the tissue sample and the like. In some embodiments, the speed is between about 0.05 mm/sec and about 10 mm/sec.

In some embodiments, the tissue cutting system further comprises a rotating device configured to cause a relative rotational movement between the tissue holder and the first cutting component from a first relative orientation to a second relative orientation, wherein in the first relative orientation, the first cutting component is configured to cut a tissue sample contained within the tissue holder in the first dimension and in the second relative orientation, the first cutting component is configured to cut the tissue sample contained within the tissue holder in the second dimension. In some embodiments, the rotating device is coupled to the tissue holder and is configured to cause a rotational movement of the tissue holder with respect to the first cutting component from a first orientation of the tissue holder to a second orientation of the tissue holder.

Some embodiments, as shown in FIG. 12A relate to a tissue cutting assembly, for producing tissue fragments of a specified size from a tissue sample. The tissue cutting assembly comprises, a tissue cutting system 1 and a filtration system 1400. In some embodiments, such as shown in FIGS. 12B-12C, the tissue cutting system comprises, i) a cutting component configured to cut a tissue sample into tissue fragments of a specified size; and ii) a reservoir configured to collect the tissue fragments. In some embodiments, the filtration system comprises a filter assembly 1410 configured to be connected to the reservoir, wherein the filter assembly comprises at least one filter unit configured to retain tissue fragments greater than the specified size, while allowing tissue fragments of the specified size to flow through. In some embodiments, the reservoir is connected to the filter assembly with the help of a connector 1450 (e.g., a tube). In some embodiments, the connector comprises a valve 1455 configured to be adjusted between an open and a closed position. In some embodiments, during the cutting operation (when the tissue sample is being cut into tissue fragments) the valve is configured to be in the closed position. In some embodiments, after the cutting operation and when the filtration system is in operation, the valve is configured to be in the open position to allow the contents of the reservoir (such as tissue fragments of the specified size and other unwanted materials) to flow into the filter assembly. In some embodiments, as shown is FIG. 12D, the filter assembly comprises i) a first filter unit 1412 of a first pore size configured to retain unwanted tissue fragments bigger than the specified size, while allowing tissue fragments of the specified size to flow through; and ii) a second filter unit 1414 of a second pore size connected in series to the first filter unit and configured to retain tissue fragments of the specified size, while allowing tissue debris and unwanted materials of smaller size to flow through. In some embodiments, the filtration system comprises a flushing mechanism (not shown) configured to supply a wash buffer at a desired flow rate for washing the filter units to remove any unwanted tissue fragments, tissue debris and/or other unwanted substances that clog the pores. In some embodiments, the filtration system further comprises a mechanical agitator for agitating the filter units to remove any unwanted tissue fragments, tissue debris and/or other unwanted substances that clog the pores. In some embodiments, the contents of the reservoir (such as tissue fragments of specified size along with other unwanted materials, such as bigger fragments, debris and other impurities) flow into the filtration system by a passive flow driven by gravity. In some embodiments, the filtration system further comprises a fluidic drive mechanism 1470 configured to be coupled to the connector and configured to drive the contents of the reservoir into the filter assembly. In some embodiments, the filtration system is an in-line filtration system (such as shown in FIG. 12B), wherein the filter assembly is connected to the reservoir while the reservoir is in its original location within the tissue cutting system. In some embodiments, the filtration system is an off-line filtration system (such as shown in FIG. 12C), wherein the reservoir (with the tissue fragments) is transferred from its original location within the tissue cutting system to the filtration system after the completion of the cutting operation. In some embodiments, the transfer of the reservoir is an automated transfer. Various mechanisms of automated transfer can be envisaged, including but not limited to conveyer mechanism, robotic arm and the like. In some embodiments, the filter system includes a rotational actuator 1475 (FIG. 3A) configured to spin the filter assembly 1410.

In some embodiments, the two or more components of the tissue cutting system are operatively connected to one another. In some embodiments, one or more components of the tissue cutting system and the overall tissue cutting assembly are controlled by a control system 2000 (e.g., a controller) (FIG. 1B). Even though a single control unit has been shown in the figure, it is to be understood that in some embodiments, there can be more than one control unit to control the various components of the tissue cutting assembly. In some embodiments, the control unit includes a processor that interfaces and/or is in electrical communication with cloud computing resources (e.g., the cloud). In some embodiments, the tissue cutting system is controllable with instructions sent and/or received over the cloud or other suitable network. In some embodiments, the tissue cutting system includes memory with software instructions stored to carry out one or more of the operations and/or methods described herein.

In some embodiments, one or more component of the tissue cutting system are disposable and/or sterilizable. In some embodiments, during operation, the tissue cutting system forms a closed system in order to maintain the sterility of the tissue sample. In some embodiments, the tissue cutting system is an automated or a semi-automated system, wherein the components of the system are configured to perform their designated functions with minimal human intervention. In some embodiments, as shown in FIGS. 3A, 3B, the tissue cutting system further comprises an emergency stop button 2010.

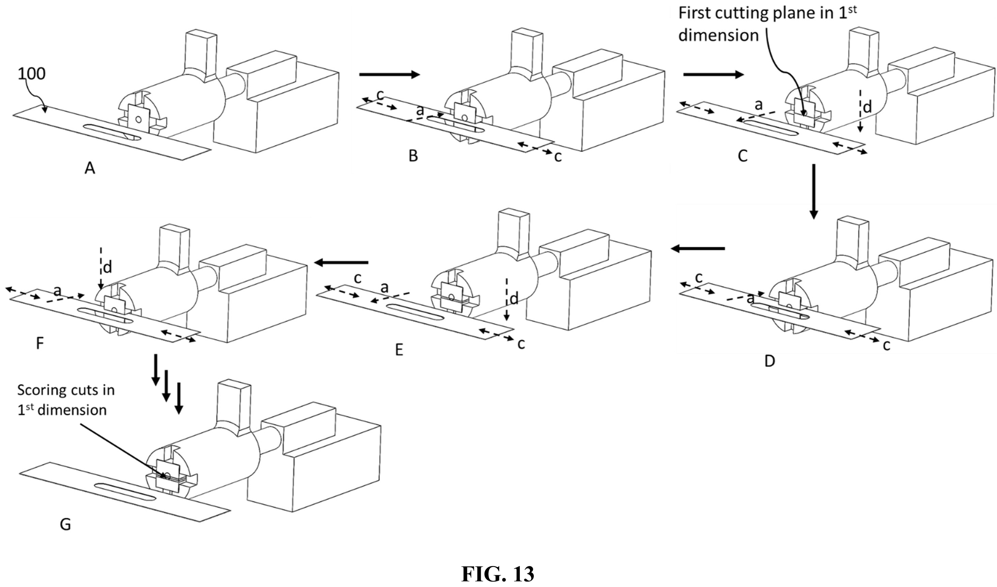

Some embodiments, such as represented in FIG. 13-19, relate to a method of cutting a tissue sample using a tissue cutting system, wherein the tissue cutting system comprises: i) a tissue holder comprising a hollow cavity defined by a sidewall, a proximal end and an opening at a distal end; ii) a first cutting component; and iii) a second cutting component. In some embodiments, the method comprises, (STEP A) preparing a tissue sample for cutting by positioning the tissue sample within the hollow cavity of the tissue holder; (STEP B) positioning the tissue holder in a first relative orientation (with respect to the first cutting component); (STEP C) operating the first cutting component and/or the tissue holder to make scoring cuts in the tissue sample in a first dimension, wherein the tissue holder is in the first relative orientation and wherein the tissue sample is contained within the hollow cavity of the tissue holder (e.g., creating relative motion between the first cutting component and the tissue holder to make scoring cuts in the tissue sample in a first dimension); (STEP D) after STEP C, rotating the tissue holder by an angle (e.g., 90 degrees) to a second relative orientation (with respect to the first cutting component); (STEP E) operating the first cutting component and/or the tissue holder to make scoring cuts in the tissue sample in a second dimension thereby producing a scored tissue sample, wherein the tissue holder is in the second relative orientation and wherein the tissue sample is contained within the hollow cavity of the tissue holder (e.g., creating relative motion between the first cutting component and the tissue holder to make scoring cuts in the tissue sample in a second dimension, thereby producing a scored tissue sample); and (STEP F) exposing a portion of the scored tissue sample through the opening at the distal end of the tissue holder; and (STEP G) driving an exposed portion of the scored tissue sample across the second cutting component (e.g., moving an exposed portion of the scored tissue sample across the second cutting component), wherein the exposed portion of the scored tissue sample is cut in a third dimension to produce tissue fragments.

In some embodiments, STEP B to STEP G comprises one cutting cycle. In some embodiments, one cutting cycle cuts only a portion of the tissue sample into tissue fragments. In some embodiments, the STEP B to STEP G are repeated to cut the entire tissue sample into tissue fragments. The "entire tissue sample" as used herein means substantially the whole tissue sample. In some embodiments, the tissue sample is a live tissue sample. In some embodiments, the first and the second cutting component are both located near the distal end of the tissue holder. In some embodiments, the first and second cutting components are mounted on a single holder (e.g., mount) at different relative positions with respect to the tissue holder, wherein the tissue sample contained within the tissue holder first makes contact with the first cutting component, wherein the first cutting component cuts the tissue sample to produce a scored tissue sample. Subsequently, the tissue holder containing the tissue sample moves towards the second cutting component, wherein the second cutting component cuts the scored tissue sample to produce tissue fragments. In some embodiments, the tissue cutting system further comprises a reservoir filled with a fluid material, wherein during STEP B to STEP G, the tissue holder containing the live tissue sample is submerged within the fluid material contained within the reservoir. In some embodiments, the method further comprises collecting the tissue fragments in the reservoir.

In some embodiments, the sidewall of the tissue holder comprises a slit. In some embodiments, the step of cutting the tissue sample while the tissue sample is contained within the hollow cavity of the tissue holder comprises, driving the first cutting component through the slit into the hollow cavity containing the tissue sample. In some embodiments, the side-wall of the tissue holder comprises a plurality of slits.

In some embodiments, the method further comprises i) moving the tissue holder to successive translational positions while in the first relative orientation, and operating the first cutting component and/or the tissue holder to make a scoring cut in the tissue sample at each translational position of the tissue holder, thereby cutting the tissue sample at successive cutting planes in the first dimension; and ii) moving the tissue holder to successive translational positions while in the second relative orientation, and operating the first cutting component and/or the tissue holder to make a scoring cut in the tissue sample at each translational position of the tissue holder, thereby cutting the tissue sample at successive cutting planes in the second dimension. In some embodiments, the translational movement of the tissue holder is caused by a translation assembly coupled to the tissue holder.

In some embodiments, the translational movement of the tissue holder is a vertical translational movement. In some embodiments, the method further comprises i) moving the tissue holder vertically to successive vertical positions while in the first relative orientation, and operating the first cutting component and/or the tissue holder to make a scoring cut in the tissue sample at each vertical position of the tissue holder, thereby cutting the tissue sample at successive cutting planes in the first dimension; and ii) moving the tissue holder vertically to successive vertical positions while in the second relative orientation, and operating the first cutting component and/or the tissue holder to make a scoring cut in the tissue sample at each vertical position of the tissue holder, thereby cutting the tissue sample at successive cutting planes in the second dimension. In some embodiments, the vertical movement of the tissue holder is caused by a vertical translation assembly coupled to the tissue holder.

In some embodiments, the method further comprises i) moving the tissue holder to successive horizontal translational positions while in the first relative orientation, and operating the first cutting component and/or the tissue holder to make a scoring cut in the tissue sample at each horizontal translational position of the tissue holder, thereby cutting the tissue sample at successive cutting planes in the first dimension; and ii) moving the tissue holder to successive horizontal translational positions while in the second relative orientation, and operating the first cutting component and/or the tissue holder to make a scoring cut in the tissue sample at each horizontal translational position of the tissue holder, thereby cutting the tissue sample at successive cutting planes in the second dimension. In some embodiments, the translational movement of the tissue holder is caused by a translation assembly coupled to the tissue holder. In some embodiments, the translation assembly comprises a x-y translation stage on which the tissue holder is mounted.

In some embodiments, the step of operating the first cutting component or the tissue holder to make scoring cuts in the tissue sample (contained within the tissue holder) comprises moving the first cutting component towards and away from the tissue sample (contained in the tissue holder) or moving the tissue holder (containing the tissue sample) towards and away from the first cutting component. In some embodiments, the step of operating the first cutting component and the tissue holder to make scoring cuts in the tissue sample (contained within the tissue holder) comprises oscillating the first cutting component while moving the tissue holder containing the tissue sample towards and away from the tissue sample.

In one example of a representative cutting cycle, the tissue holder is positioned at a first vertical position (as shown in FIG. 13A) while in the first relative orientation, and the first cutting component is moved towards the tissue sample (shown by broken arrow "a" in FIG. 13B), thereby cutting the tissue sample at a first cutting plane in the first dimension (as shown in FIG. 13C). The first cutting component is then moved backwards, away from the tissue sample to go back to the original position. Next, the tissue holder is moved vertically (upwards or downwards, such as shown by arrow "d" in FIG. 13D) to a second vertical position and the first cutting component is again moved towards the tissue sample thereby cutting the tissue sample at a second cutting plane in the first dimension (such as shown in FIG. 13E). The second cutting plane is above or below the first cutting plane depending on whether tissue holder is moved vertically downwards or upwards. In some embodiments, the first and the second cutting planes are parallel to each other. "Parallel", as used herein means substantially parallel. The vertical movement of the tissue holder, and the translational movement of the first cutting component (towards and away from the tissue sample) at each vertical position of the tissue holder, are repeated until the tissue sample is cut at successive cutting planes in a first dimension (such as shown in FIG. 13G). In some embodiments, these cuts are scoring cuts, wherein the first cutting component cuts the tissue sample, but tissue fragments (*i.e.,* fragments that detach from the tissue sample) are not produced. In some embodiments, at each cutting event during a first cutting process, the first cutting component moves into and penetrates the tissue sample to a depth dx₁, which is less than the depth of the tissue sample in the direction of the cuts.

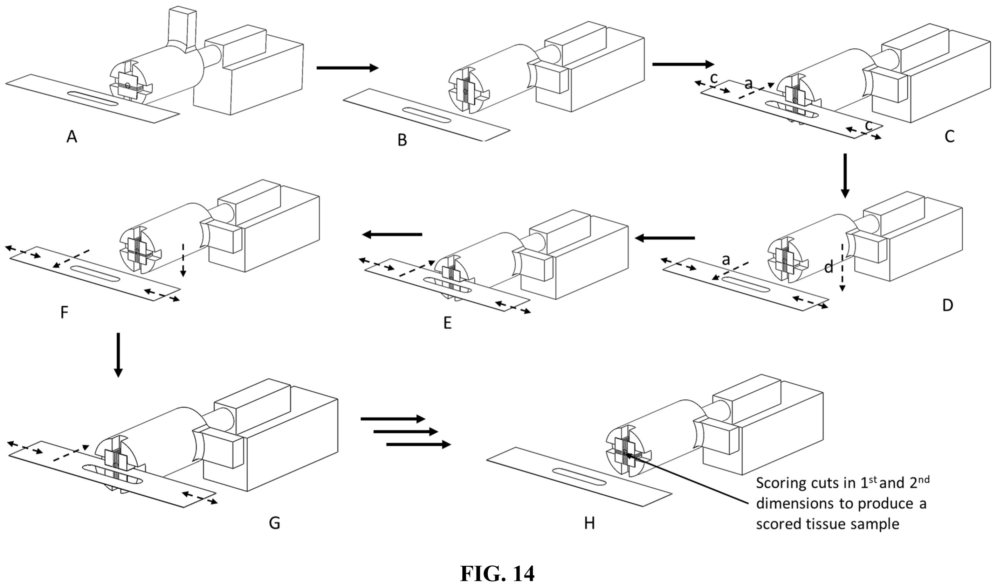

In some embodiments, after the first cutting process, the tissue holder is rotated, such as by a 90° angle, about the longitudinal axis of the tissue holder, wherein the tissue holder is rotated from a first orientation of the tissue holder (shown in FIG. 14A) to a second orientation of the tissue holder (shown in FIG. 14B). In some embodiments, the tissue holder is rotated by a rotating device coupled to the tissue holder.

In the second orientation of the tissue holder, the tissue holder is again moved to successive vertical positions (shown by arrow "d"). At each vertical position of the tissue holder, the first cutting component moves towards and away from the tissue holder (such as shown in FIGS. 14C-14G). The repeated vertical movement of the tissue holder (shown by arrow "d", and the translational movement of the first cutting component (shown by arrow "a") at each vertical position of the tissue holder, cause the tissue sample to be cut at successive cutting planes in the second dimension (such as shown in FIG. 14H). In some embodiments, these cuts are scoring cuts, wherein the first cutting component cuts the tissue sample without producing tissue fragments that detach from the tissue sample. In some embodiments, at each cutting event during the second cutting process, the first cutting component penetrates the tissue sample to a depth dy₁, which is less than the depth of the tissue sample in the cutting direction. In some embodiments, the cutting planes in the first dimension and the cutting planes in the second dimension are mutually perpendicular to each other. In some embodiments, during the first and the second cutting processes, the first cutting component is oscillated, the direction of oscillation shown by the double-sided broken arrow "c". In some embodiments, the movements "a" (scoring motion) and "c" (oscillation) are coupled and synchronous.

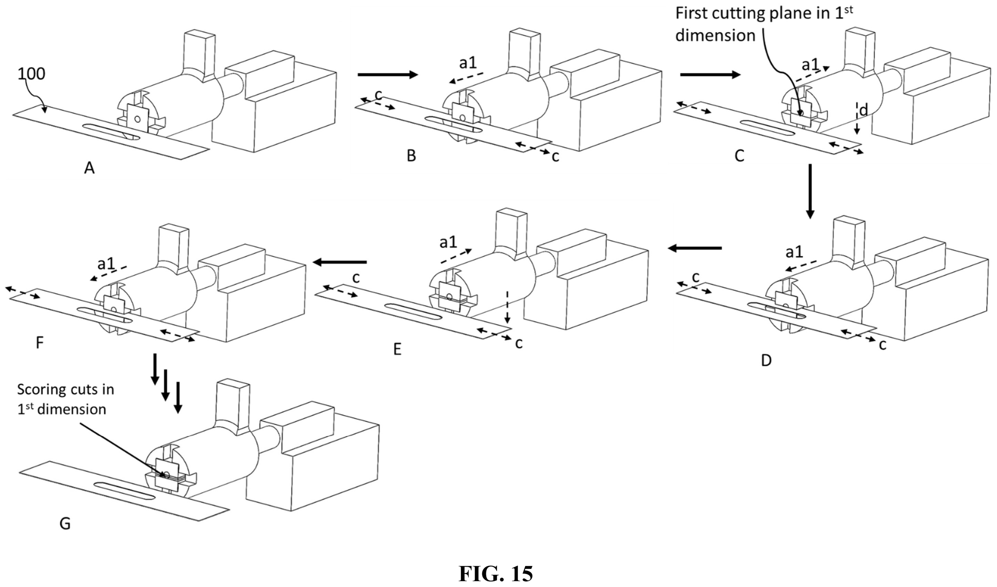

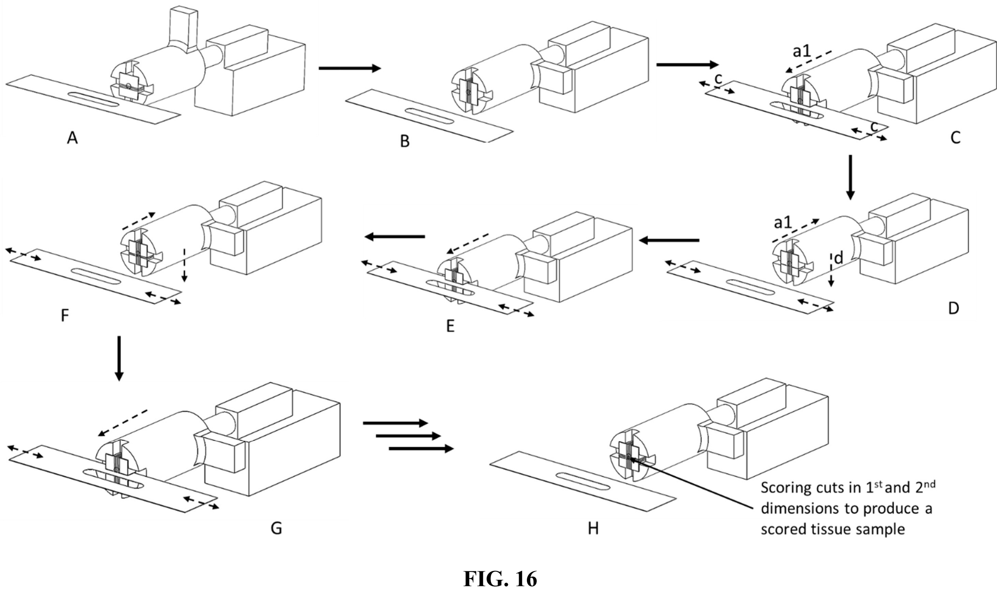

In an alternate example, the tissue holder is positioned at a first vertical position (as shown in FIG. 15A) while in the first relative orientation. At the first vertical position, the tissue holder is moved towards the first cutting component (FIG. 15B, shown by the broken arrow "a1"), causing the first cutting component to penetrate the tissue sample to a depth (dx₁), thereby cutting the tissue sample at a first cutting plane in the first dimension. The tissue holder then retracts (FIG. 15C, shown by the broken arrow "a1" in the reverse direction), moves to a second vertical position (shown by arrow "d") and the process is repeated (FIG. 15D-G), wherein the tissue sample is cut at successive cutting planes in the first dimension (FIG. 15G). The tissue holder is rotated from the first to the second relative orientation (FIG. 16A-B) and the process is repeated to cut the tissue sample at successive cutting planes in the second dimension (such as shown in FIG. 16C-H). In some embodiments, the movement of the tissue holder towards the first cutting component is caused by a translation assembly. In some embodiments, the translation assembly comprises a translation stage on which the tissue holder is mounted.

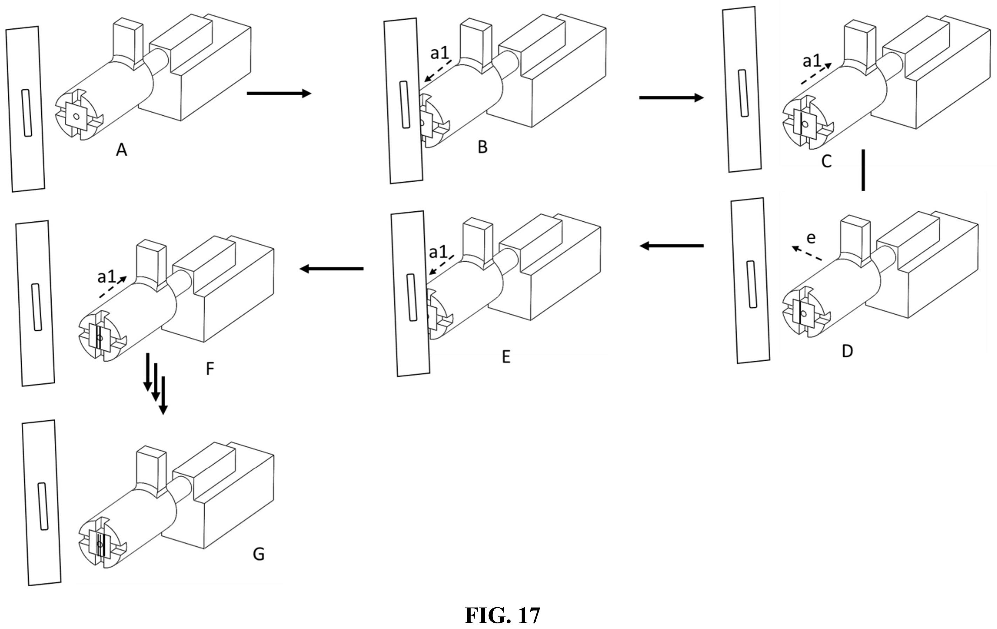

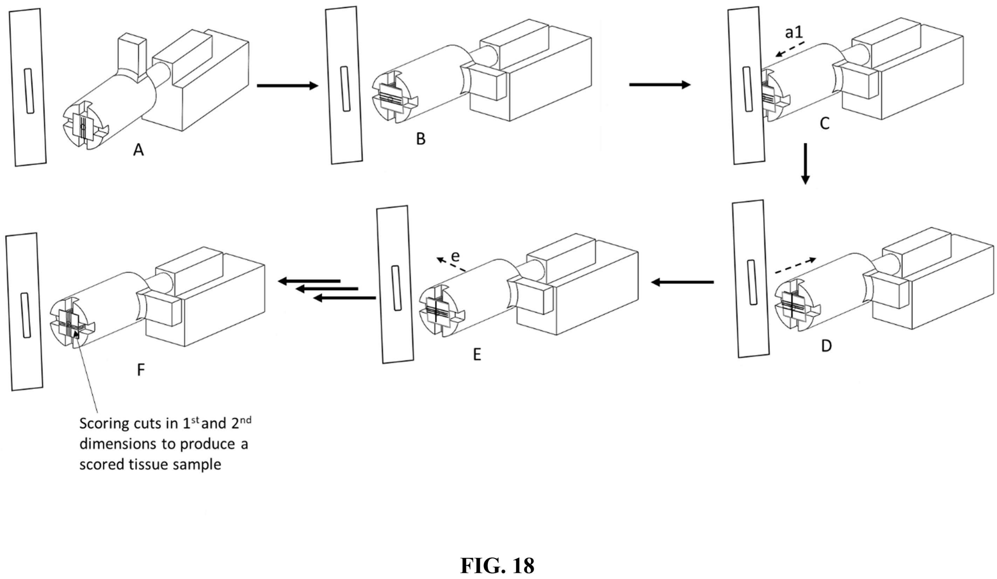

In one embodiment, as shown in FIG. 17, the tissue holder is positioned at a first horizontal position (as shown in FIG. 17A) while in the first relative orientation. At the first horizontal position, the tissue holder is moved towards the first cutting component (FIG. 17B, shown by the broken arrow "a1"), causing the first cutting component to penetrate the tissue sample to a depth (dx₁), thereby cutting the tissue sample at a first cutting plane in the first dimension. The tissue holder then retracts (FIG. 17C, shown by "a1" in the reverse direction), moves to a second horizontal position (shown by arrow "e" in FIG. 17D) and the process is repeated (FIG. 17E-17G), wherein the tissue sample is cut at successive cutting planes in the first dimension (FIG. 17G). The tissue holder is rotated from the first to the second relative orientation (FIG. 18A-18B) and the process is repeated to cut the tissue sample at successive cutting planes in the second dimension (such as shown in FIG. 18C-18F). This produces a scores tissue sample as shown in FIG. 18F. In some embodiments, the movement of the tissue holder towards the first cutting component is caused by a translation assembly. In some embodiments, the translation assembly comprises a translation stage on which the tissue holder is mounted.

In some embodiments, the step of exposing a portion of the scored tissue sample is performed with the help of a drive assembly. In some embodiments, the drive assembly drives out a portion of the scored tissue sample through the opening at the distal end of the tissue holder to expose a portion of the scored tissue sample. In some embodiments, the step of driving an exposed portion of the scored tissue sample across the second cutting component, is performed by the drive assembly and/or a linear translation assembly.

In some embodiments, after the tissue sample is cut in the first and the second dimension to produce a scored tissue sample (such as shown in FIG. 16H, 18F etc.), the tissue holder is moved towards the second cutting component. In some embodiments, the tissue holder is moved towards the second cutting component by the translation assembly comprising the translation stage. In some embodiments, the tissue holder along with the reservoir is moved towards the second cutting component. The tissue holder (optionally along with the reservoir) can be moved towards the second cutting component before, after, or during the step of exposing a portion of the scored tissue sample out of the hollow cavity of the tissue holder. In some embodiments, the tissue sample is moved towards the second cutting component without any scoring by the first cutting component. In other words, the second cutting components makes slices of the tissue sample.

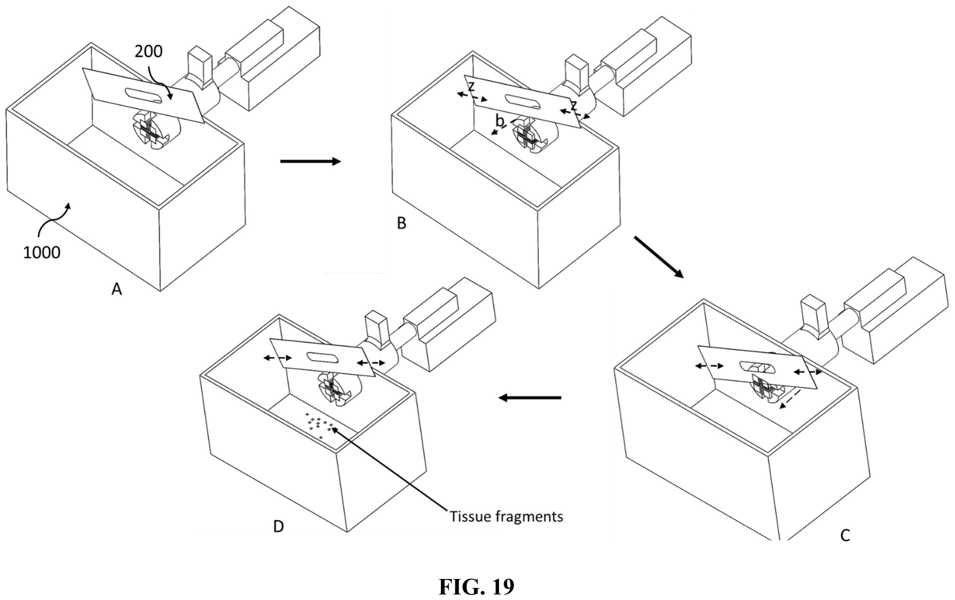

In some embodiments (such as shown in FIG. 19A-19D), after the tissue holder is positioned with respect to the second cutting component, the drive assembly drives out a portion of the scored tissue sample through the opening at the distal end of the tissue holder to expose a portion of the scored tissue sample. In some embodiments, the drive assembly drives the exposed portion of the scored tissue sample across the second cutting component to cut the exposed portion of the scored tissue in the third dimension to produce tissue fragments. As to be understood by a person of ordinary skill, even though a particular orientation of the second cutting component is depicted in FIG. 19A-19D, other orientations of the second cutting component can be envisaged. For example, the second cutting component can also be oriented vertically, such as shown in FIG. 3E, wherein β (or the angle between the linear axis of the second cutting component and the longitudinal axis of the tissue holder) is about 90°.

In some embodiments, the drive assembly drives out a portion of the scored tissue sample through the opening at the distal end of the tissue holder to expose a portion of the scored tissue sample. In some embodiments, the translation assembly drives the tissue holder (and optionally the reservoir) toward the second cutting component causing the exposed portion of the scored tissue sample to be driven across the second cutting component, wherein the exposed portion of the scored tissue sample is cut in the third dimension to produce tissue fragments. As to be understood, it is required that the exposed portion of the scored tissue sample be driven across the second cutting component in order to cut the exposed portion of the scored tissue sample in a third dimension to produce tissue fragments. The order of movements of the tissue holder towards the second cutting component (driven by the translation assembly) and the tissue sample out of the hollow cavity of the tissue holder (driven by the drive assembly), is not limiting.

The sequential events of the first, the second and the third cutting processes constitutes a cutting cycle, such as a first cutting cycle. In some embodiments, after a cutting cycle (such as a first cutting cycle), the tissue holder and the reservoir are moved back to the original position, such as by the translation assembly. In some embodiments, one cutting cycle cuts only a portion of the tissue sample into tissue fragments. In some embodiments, the first, the second and the third cutting processes are repeated for plurality of cutting cycles, wherein substantially the entire tissue sample is cut into tissue fragments.

In some embodiments, the tissue holder further comprises a sacrificial tissue-support and wherein the step of preparing the tissue sample for cutting further comprises i) positioning the tissue sample on the sacrificial tissue-support and ii) encasing the tissue sample supported on the sacrificial tissue-support with an encapsulant.

In some embodiments, one or more steps of the method of cutting the tissue sample into tissue fragments are automated or semi-automated, that is the steps are performed with minimal human intervention.

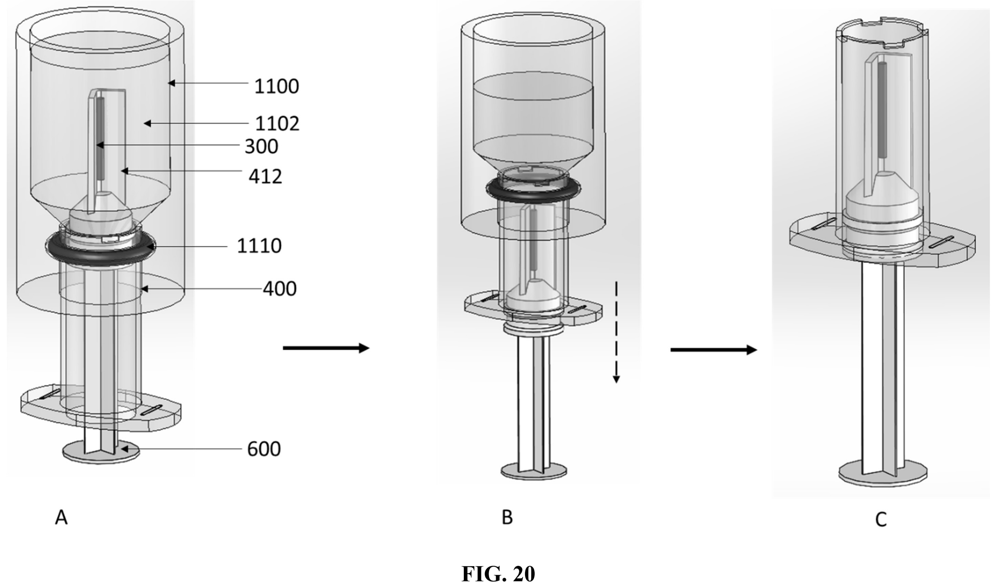

As shown in FIG. 20A-20C, some embodiments relate to a method of preparing a live tissue sample for cutting. In some embodiments, the method comprises: (STEP A) providing a tissue holder 400 comprising a hollow cavity defined by a side-wall, a proximal end, an opening at a distal end and a sacrificial tissue-support; (STEP B) positioning the live tissue sample 300 securely on a portion of the sacrificial tissue-support 412, wherein said portion of the sacrificial tissue-support is exposed out of the hollow cavity through the opening at the distal end of the tissue holder; (STEP C) coupling an encapsulant-reservoir 1100 comprising an internal volume 1102 filled with an encapsulant precursor, to the opening at the distal end of the tissue holder, wherein the sacrificial tissue-support supporting the live tissue sample extends into the internal volume of the encapsulant-reservoir filled with the encapsulant precursor (such as shown in FIG. 20A); and (STEP D) retracting the sacrificial tissue-support supporting the live tissue sample into the hollow cavity of the tissue holder, wherein the encapsulant precursor is drawn into the hollow cavity along with the live tissue sample (such as shown in FIG. 20B). In some embodiments, the method further comprises, subjecting the encapsulant precursor to gelation condition to produce an encapsulant 1104 after STEP D, wherein the live tissue sample (which is supported on the sacrificial tissue-support within the hollow cavity of the tissue holder), is encased by the encapsulant; and detaching the encapsulant-reservoir from the tissue holder (such as shown in FIG. 20C).

In some embodiments, the step of positioning the live tissue sample securely on a portion of the sacrificial tissue-support comprises attaching the live tissue sample to a portion of the sacrificial tissue-support using an adhesive. In some embodiments, the step of retracting the sacrificial tissue-support supporting the live tissue sample into the hollow cavity of the tissue holder is performed with the help of a drive assembly, wherein the drive assembly is coupled to the sacrificial tissue-support.

Some embodiments relate to a kit for preparing a tissue sample for cutting, comprising: (a) a tissue holder comprising a hollow cavity defined by a side-wall, a proximal end, an opening at a distal end, and a sacrificial tissue-support. The sacrificial tissue-support is configured to support the tissue sample. In some embodiments, the sacrificial tissue-support is configured to be driven out of and retracted into the hollow cavity. The sacrificial tissue-support is configured to be cut by a cutting component. In some embodiments, the kit further comprises (b) an encapsulant-reservoir containing an encapsulant precursor within an internal volume of the encapsulant-reservoir, wherein the encapsulant-reservoir is configured to be detachably coupled to the opening at the distal end of the tissue holder thereby forming an interface, which allows the flow of the encapsulant precursor from the internal volume of the encapsulant-reservoir into the hollow cavity of the tissue holder. In some embodiments, the sacrificial tissue-support is configured to be driven out of and retracted into the hollow cavity with the help of a drive assembly coupled to the sacrificial tissue-support. In some embodiments, the kit is disposable. In some embodiments, the kit is for preparing a live tissue sample for cutting. In some embodiments, the encapsulant precursor is an alginate solution.

In some embodiments, the sidewall of the tissue holder comprises a slit, wherein the slit permits a cutting component to pass through and cut a tissue sample contained within the hollow cavity of the tissue holder, wherein the width of the slit is between about 1 mm and about 10 mm and wherein the depth of the slit is between about 50 µm and about 1000 µm. In some embodiments, the sidewall of the tissue holder comprises a plurality of slits, wherein the spacing between two adjacent slits in the plurality of slits is between about 300 µm and about 2000 µm. In some embodiments, the sacrificial tissue-support comprises a non-flat surface comprising a groove.

Some embodiments relate to an operational assembly for cutting a live tissue sample into tissue fragments, comprising: (a) a tissue holder comprising a hollow cavity defined by a side-wall, a proximal end, an opening at a distal end, and a sacrificial tissue-support within the hollow cavity of the tissue holder and (b) a live tissue sample positioned on the sacrificial tissue-support within the hollow cavity, wherein the sacrificial tissue-support supporting the live tissue sample is configured to be driven out of the hollow cavity to expose a portion of the live tissue sample through the opening at the distal end. In some embodiments, the assembly further contains, (c) a first cutting component configured to cut the live tissue sample by scoring cuts in a first dimension and a second dimension respectively to produce a scored tissue sample, wherein the first cutting component is configured to cut the live tissue sample while the live tissue sample is contained within the hollow cavity of the tissue holder; and (d) a second cutting component configured to cut the scored tissue sample in a third dimension to produce tissue fragments, wherein the second cutting component is configured to cut an exposed portion of the scored tissue sample by cutting through the sacrificial tissue-support. In some embodiments, the assembly further contains a reservoir filled with a fluid material, wherein a portion of the tissue holder containing the live tissue sample is submerged within the fluid material and wherein the reservoir is configured to receive the tissue fragments. As used herein, an exposed portion of the scored tissue sample is a portion of the scored tissue sample that is exposed out of the hollow cavity of the tissue holder, such as through the opening at the distal end. In some embodiments, the exposed portion of the scored tissue sample is supported on the sacrificial tissue-support. In some embodiments, the operation assembly is a closed system. In some embodiments, the operational assembly or a component thereof is maintained at a temperature between about 1 °C and about 10 °C.

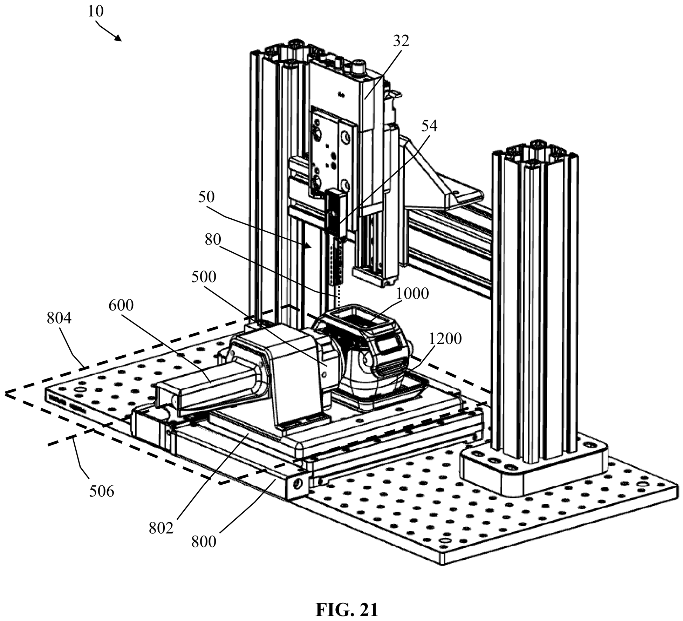

With reference to FIGS. 21-30, a tissue cutting system 10 is illustrated. The issue cutting system 10 includes a tissue holder 400, a first cutting component 100 (e.g., a first blade), a second cutting component 200 (e.g., a second blade), a rotating device 500, a drive assembly 600, a translation assembly 800, a reservoir 1000, and a nest 1200. The tissue holder 400 includes a hollow cavity 406 and is configured to hold a tissue sample 300 (e.g., a live tissue sample, a biopsy, an excision, etc.) within the hollow cavity 406.

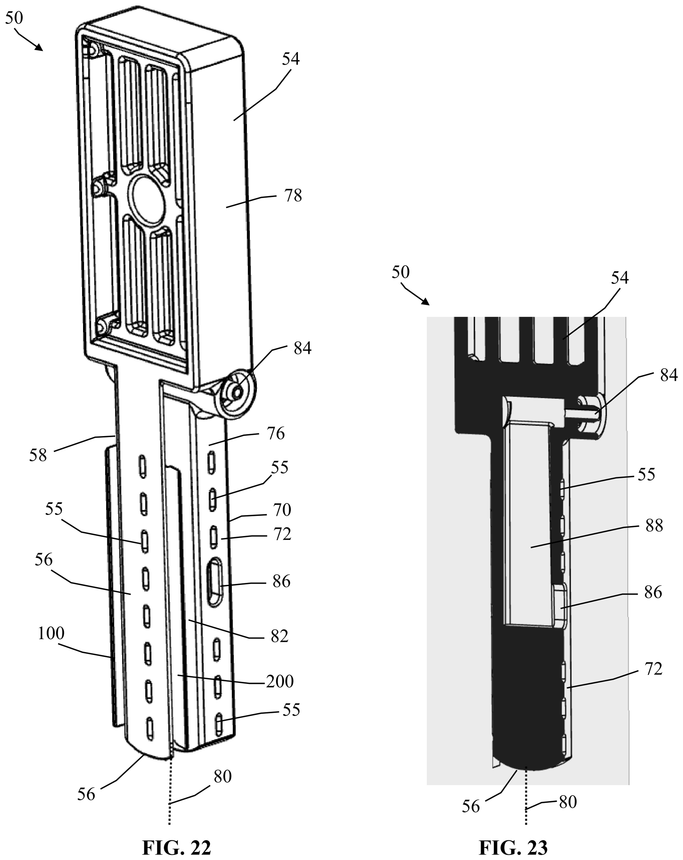

With reference to FIGS. 22-25, a cutting assembly 50 includes a mount 54, the first cutting component 100, and the second cutting component 200. The mount 54 includes a first surface 56 (e.g., a front surface). In the illustrated embodiment, the first surface 56 is facing the tissue holder 400. The mount 50 further includes a first mount surface 58 (e.g., a side surface) that intersects the first surface 56 at a first angle 60. In the illustrated embodiment, the first angle 60 is approximately 90 degrees. The mount 54 further includes a second mount surface 62 (e.g., an interior surface) that intersects the first surface 56 at a second angle 64. In some embodiments, the second angle 64 is within a range of approximately 1 degree to approximately 20 degrees. In the illustrated embodiment, the second angle 64 is approximately 12.5 degrees. In the illustrated embodiment, the mount 54 includes a plurality of visual indicia 55 to aid in, for example, system alignment.

With continued reference to FIGS. 22-25, the first cutting component 100 is coupled to the first mount surface 58, and the second cutting component 200 is coupled to the second mount surface 62. In the illustrated embodiment, the mount 54 includes a first seat 66 extending from the first mount surface 58 and a second seat 68 extending from the second mount surface 62. The first cutting component 100 abuts the first seat 66 and the second cutting component 200 abuts the second seat 68. In other words, the cutting components 100, 200 are secured against the respective seats 66, 68. In some embodiments, an adhesive is utilized to secure the cutting components to the respective mount surfaces. A first cutting plane 102 of the first cutting component 100 intersects a second cutting plane 202 of the second cutting component 200 at an angle 150. In the illustrated embodiment, the angle 150 is approximately 77.5 degrees.

As detailed herein, the first cutting component 100 is configured to cut a tissue sample contained within the tissue holder 400 by scoring cuts in a first dimension and in a second dimension respectively, to produce a scored tissue sample. In particular, the first cutting component 100 is configured to protrude into the hollow cavity 406 and cut the tissue sample 300 while the tissue sample 300 is contained within the hollow cavity 406. The second cutting component 200 is configured to cut the scored tissue sample in a third dimension to produce tissue fragments.

With continued reference to FIGS. 22-25, the mount 54 further includes a second surface 70 (e.g., a rear surface), a third surface 72 (e.g., a side surface), and a fourth surface 74 (e.g., an internal surface). In the illustrated embodiment, the second surface 70 extends approximately parallel to the first surface 56, and the second surface 70 intersects the first mount surface 58 at approximately 90 degrees. In the illustrated embodiment, the second surface 70 faces away from the tissue holder 400. The third surface 72 extends approximately parallel to the first mount surface 58, and the third surface72 intersects the second surface 70 at approximately 90 degrees. The fourth surface 74 extends approximately parallel to the first surface 56, and the fourth surface 74 intersects the third surface 72 at approximately 90 degrees. In the illustrated embodiment, the surfaces 56, 58, 62, 70, 72, 74 are formed on a finger portion 76 that extends from a securing body portion 78. In the illustrated embodiment, the finger portion 76 extends from the securing body portion 78 along a mount axis 80. In some embodiments, the securing body portion 78 is coupled to a linear rail, actuator, oscillator, etc. with a fastener.

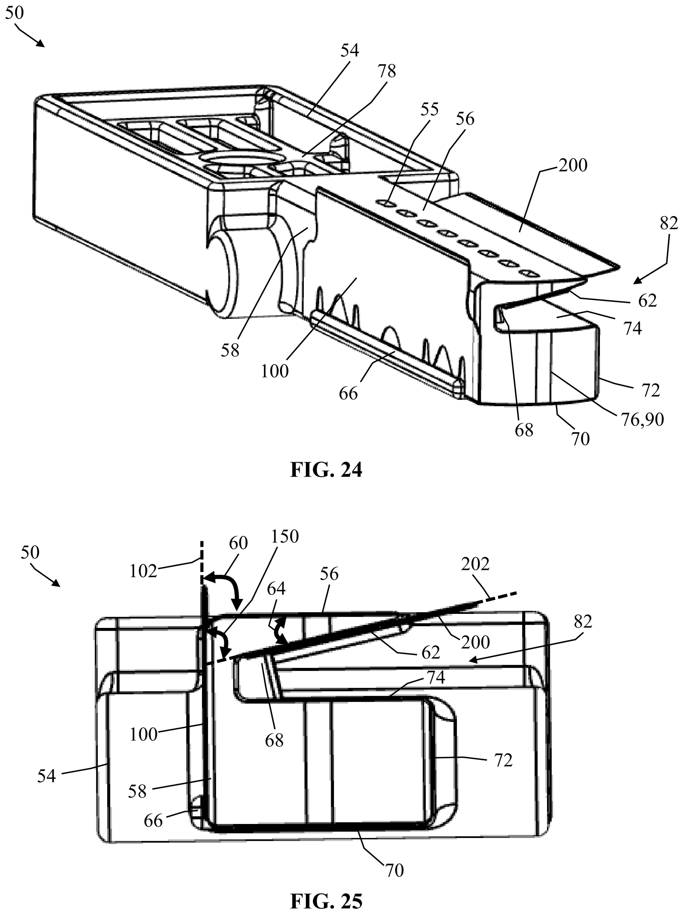

With continued reference to FIGS. 24 and 25, the mount 54 includes a notch 82 formed in the finger portion 76. In the illustrated embodiment, the notch 82 is at least partially formed by the second mount surface 62 and the fourth surface 74. In other words, the notch 82 defines an internal cavity or space in the mount 54 that receives the second cutting component 200. Advantageously, the notch 82 provides a space for tissue fragments to go during a cutting process with the second cutting component 200. In other words, the tissue fragments cut by the second cutting component 200 can slide into the notch 82 to improve cut quality.

With reference to FIGS. 22 and 23, the mount 54 includes a fluid input port 84, an outlet 86, and a passageway 88 extending between the fluid input port 84 and the outlet 86. The passageway 88 extends parallel to the first surface 56 and the first mount surface 58 and extends along the mount axis 80. In some embodiments, the fluid input port 84 is configured to receive a tube fluidly coupled to a fluid source (e.g., an oxygen source). As such, oxygen supplied to the mount 54, travels through the passageway 88 and exits the outlet 86, thereby entering the reservoir 1000. In some embodiments, a plug including a plurality of pores is positioned within the outlet 86 to aid in dispersion of the fluid exiting the outlet 86. The mount 54 therefore includes at least a portion of an oxygenation unit 1300. In the illustrated embodiment, the outlet 86 is positioned on the third surface 72 and is spaced from a distal end 90 of the finger portion 76. In some embodiments, the outlet 86 is positioned at the distal end 90 or any other suitable location on the finger portion 76.

With reference to FIG. 21, the cutting assembly 50 is coupled to the oscillator 32 (e.g., a voice coil actuator). The oscillator 32 is configured to move (e.g., oscillate) the first cutting component 100 and the second cutting component 200 along the mount axis 80. In some embodiments, the oscillator 32 is configured to control a frequency and an amplitude of movement for the cutting assembly 50. In some embodiments, the frequency is within a range of approximately 20 Hz to approximately 200 Hz.

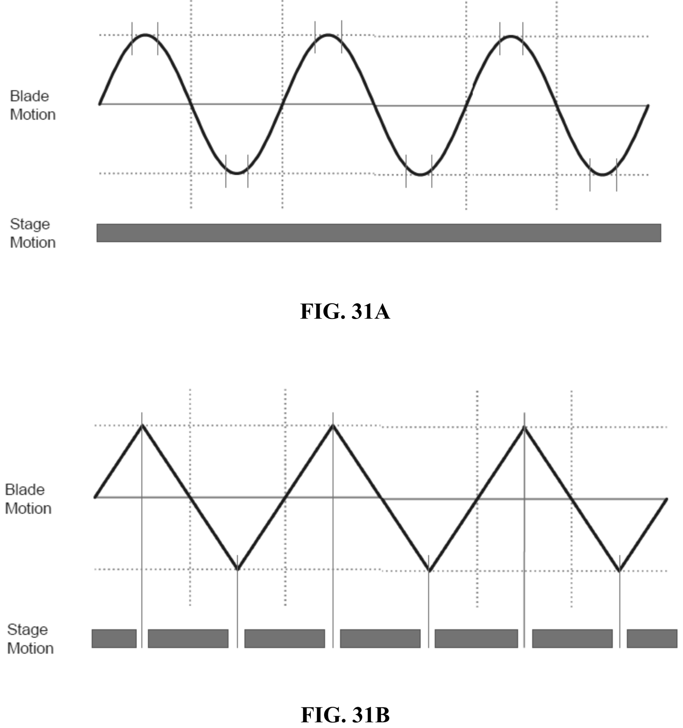

With reference to FIG. 31A, in one embodiment, the cutting assembly 50 is controlled with a sin wave oscillation ("blade motion"). Vertical lines on the blade motion curve indicate when the cutting assembly 50 motion is near zero or zero. In this example, the stage motion (e.g., motion of the tissue sample feeding into the cutting component) is continuous. As the cutting assembly 50 with the cutting components 100, 200 are slowing down or at zero, the tissue sample 300 continues to move into the cutting components 100, 200, referred to herein as "plunging."

With reference to FIG. 31B, in another embodiment, the cutting assembly 50 is control with a triangle wave oscillation ("blade motion"). In some embodiments, the oscillation is a sawtooth wave. Vertical lines on the blade motion curve indicate when the cutting assembly 50 motion is near zero or zero. In this example, the stage motion is only energized when the cutting assembly 50 is moving a constant velocity. In other words, the tissue sample 300 moves toward the cutting component 100, 200 only when the cutting component 100, 200 is moving with a constant velocity. In this example, there is no "plunging" of the tissue sample into a motionless cutting component because the stage motion and tissue sample feed pauses when the cutting component is changing directions. Advantageously, removing plunging of the tissue sample into a motionless blade improves overall cut time and increased control over individual cut parameters. In some embodiments, the cut parameters are, for example, blade stroke, blade velocity, cut ratio, feed distance, and/or feed speed.

As detailed herein, a method of cutting a tissue sample using a tissue cutting system with a cutting component comprises (STEP A) moving the cutting component cyclically or periodically at a constant velocity in a first direction and at a constant velocity in a second direction opposite the first direction ("blade motion" of FIG. 31B); and (STEB B) moving the tissue sample toward the cutting component when the cutting component is moving at the constant velocity in the first direction or the second direction ("stage motion" of FIG. 31B). In some embodiments, moving the tissue sample toward the cutting component creates scoring cuts in the tissue sample. In other embodiments, moving the tissue sample toward the cutting component creates tissue fragments (e.g., cubes, slices, etc.).

In some embodiment, moving the tissue sample relative to the cutting component includes rotating the tissue sample relative to the cutting component (e.g., a "spin cut"). The spin cut is an alternative cutting method. With a spin cut method, the rotational stage is be employed to spin the tissue sample, providing relative tissue sample motion to a stationary cutting component. In other embodiment, the otherwise stationary cutting component makes a single long stroke, utilizing the full length of the cutting surface while the tissue sample spins against the cutting component. Given the sample cut rate is *RPM x radius of sample remaining* and the radius decreases as the sample is being cut. As such, the rotational velocity increases to maintain a consistent cut rate. When the radius approaches zero, a hybrid approach of using the sawtooth or triangle cut profile motion to finish the cut be implemented.

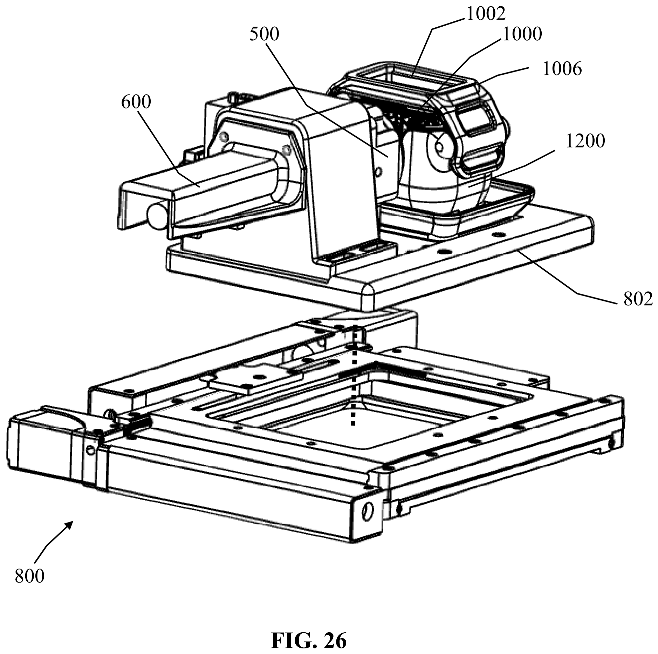

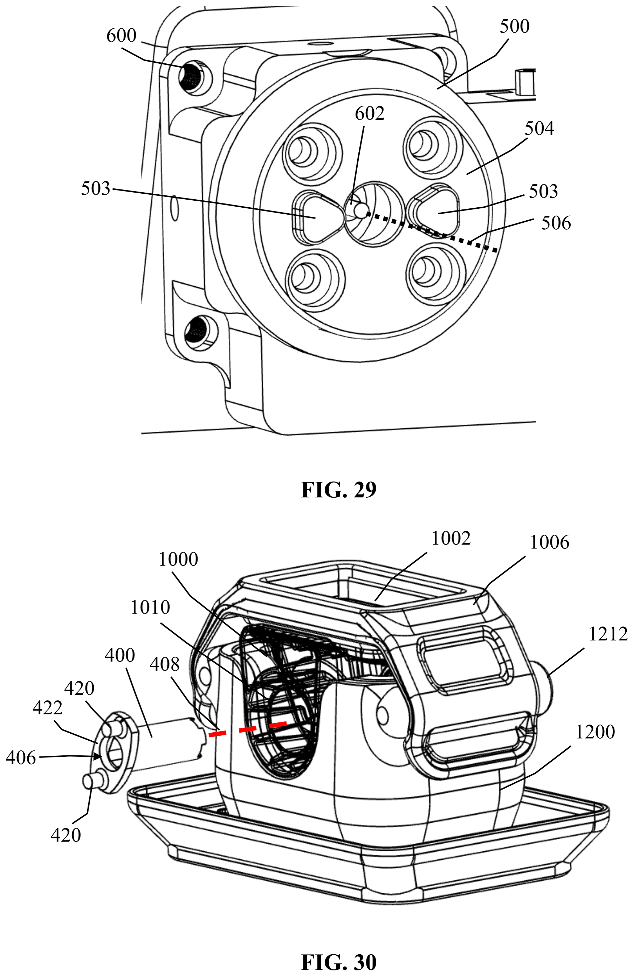

With reference to FIGS. 26-29, the rotating device 500 is configured to cause relative rotational movement between the tissue holder 400 and the first cutting component 100. In other words, the rotating devices 500 moves the tissue holder 400 and/or the first cutting component 100 between a first relative orientation and a second relative orientation. When in the first relative orientation, the first cutting component 100 is configured to cut the tissue sample within the tissue holder 400 in the first dimension. When in the second relative orientation, the first cutting component 100 is configured to cut the tissue sample contained within the tissue holder 400 in the second dimension. In the illustrated embodiment, the rotating device 500 includes at least one protrusion 503 extending from an axial end face 504 of the rotating device 500 (FIG. 29). The protrusions 503 are configured to rotate about a longitudinal axis 506 in response to energization of the rotating device 500. In the illustrated embodiment, the longitudinal axis 408 of the tissue holder 400 is aligned with the longitudinal axis 506 of the rotating device 500 during operation. The rotating protrusions 503 of the rotating device 500 are configured to engage and drive corresponding protrusions 420 formed on a rear axial surface 422 of the tissue holder 400 (FIG. 30), thereby causing rotation of the tissue holder 400 about the axis 408, 506. In other words, the rotating device 500 is coupled to the tissue holder 400 and is configured to rotate the tissue holder 400 about the longitudinal axis 408, 506 between the first relative orientation and the second relative orientation. In some embodiments, the rotating device rotates the tissue holder about the longitudinal axis by approximately 90 degrees.

During operation, the cutting assembly 50 is at least partially received within the reservoir 1000. The reservoir 1000 includes an internal space 1002 (e.g., a cavity, a bowl, a bucket) and a fluid material is positioned within the internal space 1002. The reservoir 1000 is configured to collect the tissue fragments within the internal space 1002. In the illustrated embodiment, the internal space 1002 is at least partially defined by a sidewall 1004. In the illustrated embodiment, a lid 1006 is pivotably coupled to the sidewall 1004 and movable between an open and closed configuration. In the illustrated embodiment, the mount 54 of the cutting assembly 50 is coupled to an oxygen source, the oxygen source is fluidly coupled to the reservoir 1000 when the cutting assembly 500 is submerged in the fluid material and is configured to oxygenate the fluid material in the internal space 1002 of the reservoir 1000.

In the illustrated embodiment, at least a portion of the tissue holder 400 protrudes into the internal space 1002 of the reservoir 1000 and is submerged within the fluid material. In the illustrated embodiment, the tissue holder 400 is at least partially received within an aperture 1010 (e.g., an opening) formed in the sidewall 1004 (FIG. 30). In the illustrated embodiment, the aperture 1010 is aligned with the axis 506 of the rotating device 500.

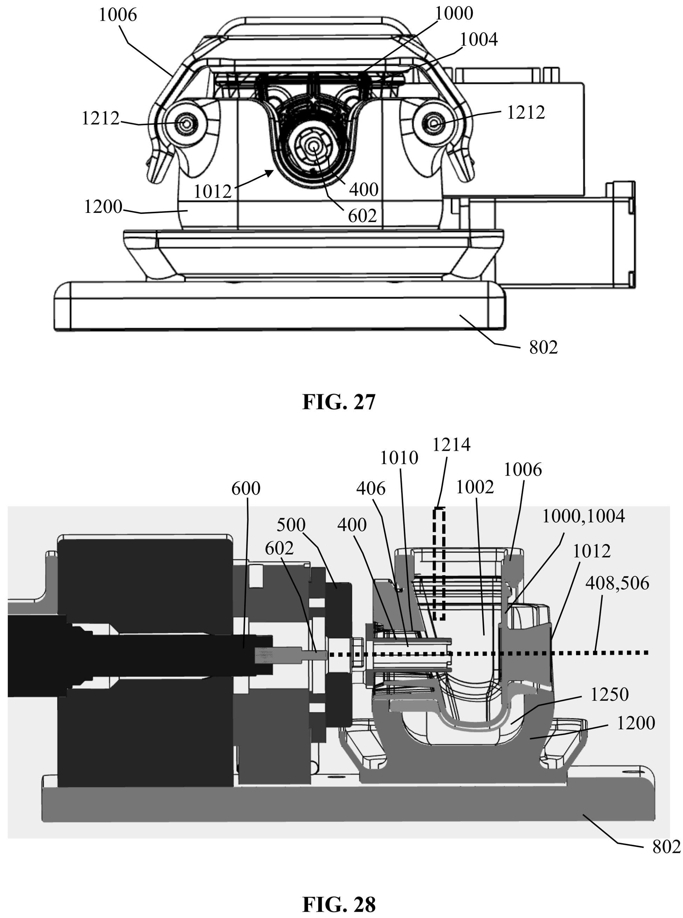

With reference to FIG. 27, in some embodiments, the reservoir 1000 includes a window 1012 (e.g., optical port) in the sidewall 1004 positioned opposite the aperture 1010. The window 1012 enables visualization of the tissue holder 400 and the tissue sample 300 submerged in the fluid material in the reservoir 1000 during operation. In some embodiments, the window 1012 is configured to prevent fogging during temperature control (e.g., chilling) of the reservoir 1000.

With reference to FIG. 28, the drive assembly 600 is coupled to the tissue sample 300. The drive assembly 600 includes a drive rod 602 that is configured to translate along the axis 506. During operation, the drive rod 602 is at least partially received within the tissue holder 400. A portion of the scored tissue sample is exposed from the tissue holder 400 in response to activation of the drive assembly 600. In other words, the drive rod 602 pushes a portion of the tissue sample out of the tissue holder 400. Then, the second cutting component 200 is configured to cut the exposed portion of the score tissue sample.

With continued reference to FIG. 28, the nest 1200 is configured to hold the reservoir 1000 and to provide temperature control of the reservoir 1000. The nest 1200 includes an internal cavity 1250 (e.g., enclosed space) for circulating a heat exchange fluid through the nest 1200. In other words, the nest 1200 and heat exchange fluid provide temperature control of the reservoir 1000. In the illustrated embodiment, the nest 1200 includes ports 1212 (e.g., inlet and outlet) in fluid communication with the internal cavity 1250. The ports 1212 are configured to couple to a tubing, for example, to receive and return the heat exchange fluid. In some embodiments, the heat exchange fluid is circulated through the nest to maintain the temperature of the reservoir 1000 (and the fluid material in the reservoir 1000) within a range of approximately 1°C and approximately 10°C. In other embodiments, the heat exchange fluid is circulated through the nest to maintain the temperature of the reservoir 1000 (and the fluid material in the reservoir 1000) within a range of approximately 35°C and approximately 38°C.

As detailed herein, in some embodiments, the nest 1200 is fluidly coupled to an external heat exchanger. In some embodiments, the heat exchanger is configured pump the heat exchange fluid through the cavity 1210 in the nest 1200. In some embodiments, a temperature sensor 1214 is provided and configured to detect the temperature of the fluid material in the reservoir 1000. In some embodiments, the temperature sensor 1214 is submerged within the fluid material. In other embodiments, the temperature sensor 1214 is spaced from the fluid material. In some embodiments, the temperature sensor 1214 is coupled to the lid 1006 on the reservoir 1000 that moves between open and closed configurations.

With reference to FIG. 26, the translation assembly 800 is coupled to the tissue holder 400 and configured to move the tissue holder 400 and tissue sample 300 with respect to the first cutting component 100 and the second cutting component 200. In the illustrated embodiment, the translation assembly 800 includes a translation stage 802 (e.g., a platform) that supports the reservoir 1000, the nest 1200, the tissue holder 400, the rotating device 500, and the drive assembly 600. The translation stage 802 is configured to cause a translation motion of the tissue holder 400 towards and away from the first cutting component 100 and/or the second cutting component 200. In some embodiments, the tissue holder 400 translates toward the cutting components 100, 200 at a predetermined speed. In the illustrated embodiment, the translation assembly 800 is configured to move the translation stage 802 in a horizontal X-Y translation plane 804 (FIG. 21). During operation, the mount axis 80 is approximately perpendicular to the translation plane 804. In the illustrated embodiment, the axis 408 of the tissue holder 400 and the axis 506 of the rotating device 500 and drive assembly 600 translate as the stage 802 moves within the translation plane 804.

The following embodiments are of particular interest:
1. A tissue cutting system comprising:
   a tissue holder with a hollow cavity, wherein the tissue holder is configured to hold a tissue sample within the hollow cavity;
   a first cutting component configured to cut a tissue sample contained within the hollow cavity of the tissue holder by scoring cuts in a first dimension and in a second dimension respectively, to produce a scored tissue sample;
   a rotating device configured to cause a relative rotational movement between the tissue holder and the first cutting component, between a first relative orientation and a second relative orientation,
   wherein in the first relative orientation, the first cutting component is configured to cut the tissue sample contained within the tissue holder in the first dimension and in the second relative orientation, the first cutting component is configured to cut the tissue sample contained within the tissue holder in the second dimension; and
   a second cutting component configured to cut the scored tissue sample in a third dimension to produce tissue fragments.
2. The tissue cutting system of embodiment 1, further comprising a drive assembly coupled with the tissue sample, a portion of the scored tissue sample is exposed at an opening at a distal end of the tissue holder in response to activation of the drive assembly.
3. The tissue cutting system of embodiment 2, wherein the second cutting component is configured to cut an exposed portion of the scored tissue sample.
4. The tissue cutting system of embodiment 1, wherein the tissue holder further comprises a sacrificial tissue-support, wherein the sacrificial tissue-support is configured to support the tissue sample, and wherein the second cutting component is configured to cut the tissue sample by cutting through the sacrificial tissue-support.
5. The tissue cutting system of embodiment 4, wherein the sacrificial tissue-support includes a non-flat surface with a groove, wherein the groove is configured to support the tissue sample.
6. The tissue cutting system of embodiment 1, further comprising a translation assembly coupled to the tissue holder and configured to move the tissue holder containing the tissue sample with respect to the first cutting component and/or the second cutting component.
7. The tissue cutting system of embodiment 6, wherein the translation assembly comprises a translation stage on which the tissue holder is mounted, wherein the translation stage is configured to cause a translation motion of the tissue holder towards and away from the first cutting component and/or the second cutting component.
8. The tissue cutting system of embodiment 1, wherein a sidewall of the tissue holder comprises a slit, wherein the slit permits the first cutting component to pass through and cut the tissue sample while the tissue sample is contained within the hollow cavity of the tissue holder.
9. The tissue cutting system of embodiment 8, wherein the slit is one of a plurality of slits, and wherein each of the plurality of slits permits the first cutting component to pass through and cut the tissue sample while the tissue sample is contained within the hollow cavity of the tissue holder.
10. The tissue cutting system of embodiment 9, wherein two adjacent slits of the plurality of slits are positioned apart within a range of 300 µm to 2000 µm.
11. The tissue cutting system of embodiment 8, wherein a width of the slit is within a range of 1 mm to 10 mm.
12. The tissue cutting system of embodiment 8, wherein a depth of the slit is within a range of 50 µm to 1000 µm.
13. The tissue cutting system of embodiment 1, wherein the first cutting component is configured to protrude into the hollow cavity through an opening at a distal end of the tissue holder and cut the tissue sample while the tissue sample is contained within the hollow cavity.
14. The tissue cutting system of embodiment 1, further comprising a reservoir with an internal space, and a fluid material positioned within the internal space; wherein at least a portion of the tissue holder protrudes into the internal space of the reservoir and is submerged within the fluid material, and wherein the reservoir is configured to collect the tissue fragments within the internal space.
15. The tissue cutting system of embodiment 14, further comprising an oxygenation unit including an oxygen source, wherein the oxygen source is coupled to the reservoir and is configured to oxygenate the fluid material in the internal space of the reservoir.
16. The tissue cutting system of embodiment 14, further comprising a nest configured to hold the reservoir, wherein the nest comprises a passageway for circulating a heat exchange fluid through the nest.
17. The tissue cutting system of embodiment 16, wherein the heat exchange fluid circulated through the nest maintains the temperature of the reservoir and the fluid material in the reservoir within a range of 1 °C to 10 °C.
18. The tissue cutting system of embodiment 16, wherein the heat exchange fluid circulated through the nest maintains the temperature of the reservoir and the fluid material in the reservoir within a range of 35 °C to 38°C.
19. The tissue cutting system of embodiment 16, further comprising a heat exchanger configured to pump the heat exchange fluid through the passageway in the nest, and further comprising a temperature sensor configured to detect the temperature of the fluid material in the reservoir.
20. The tissue cutting system of embodiment 14, further comprising a filter assembly attachable to the reservoir, wherein the filter assembly comprises at least a first filter unit configured to retain tissue fragments larger than a specified size.
21. The tissue cutting system of embodiment 20, wherein the filter assembly further comprises at least a second filter unit connected in series with the first filter unit, wherein the second filter unit is configured to retain tissue fragments of the specified size.
22. The tissue cutting system of embodiment 1, wherein the rotating device is coupled to the tissue holder and is configured to rotate the tissue holder about a longitudinal axis between the first relative orientation and the second relative orientation.
23. The tissue cutting system of embodiment 1, wherein the rotating device rotates the tissue holder about the longitudinal axis by an angle of 90°.
24. The tissue cutting system of embodiment 1, further comprising an oscillator coupled to the first cutting component and the second cutting component; the oscillator configured to oscillate the first cutting component and the second cutting component at a frequency within a range of 20 Hz to 200 Hz.
25. The tissue cutting system of embodiment 24, further comprising a mount, wherein the first cutting component and the second cutting component are coupled to the mount, wherein a first cutting plane of the first cutting component intersects a second cutting plane of the second cutting component at an angle.
26. The tissue cutting system of embodiment 1, wherein the tissue sample is a live tissue sample.
27. A tissue cutting system comprising:
   a tissue holder with a hollow cavity, a proximal end, an opening at a distal end, and a sacrificial tissue-support positioned within the hollow cavity;
   wherein the sacrificial tissue-support is configured to support a tissue sample, and wherein the sacrificial tissue-support is configured to be driven out of the hollow cavity to expose a portion of the tissue sample through the opening at the distal end;
   a cutting component configured to cut the tissue sample to produce tissue fragments, wherein the cutting component is configured to cut an exposed portion of the tissue sample by cutting through the sacrificial tissue-support; and
   a reservoir filled with a fluid material, wherein a portion of the tissue holder is submerged within the fluid material.
28. The tissue cutting system of embodiment 27, wherein the sacrificial tissue-support includes a non-flat surface with a groove, wherein the groove is configured to support the tissue sample.
29. The tissue cutting system of embodiment 27, wherein the sacrificial tissue-support includes a non-flat surface with a plurality of grooves, wherein each groove in the plurality of grooves is configured to support a tissue sample for simultaneous cutting of multiple tissue samples.
30. A tissue cutting system comprising:
   a tissue holder configured to hold a tissue sample;
   a cutting assembly including a mount, a first cutting component coupled to the mount, a second cutting component coupled to the mount, and an oscillator coupled to the mount, wherein the oscillator is configured to move the first cutting component and the second cutting component, wherein the first cutting component is configured to cut the tissue sample in a first dimension and a second dimension to produce a scored tissue sample, and wherein the second cutting component is configured to cut the scored tissue sample in a third dimension to produce tissue fragments;
   a rotating device configured to cause a relative rotational movement between the tissue holder and the first cutting component between a first relative orientation and a second relative orientation, wherein in the first relative orientation the first cutting component is configured to cut a tissue sample contained within the tissue holder in the first dimension and in the second relative orientation the first cutting component is configured to cut the tissue sample contained within the tissue holder in the second dimension; and
   a translation stage, wherein the tissue holder is coupled to the translation stage, and the tissue holder translates with respect to the first cutting component and the second cutting component in response to activation of the translation stage.
31. The tissue cutting system of embodiment 30, wherein the tissue holder translates toward the first cutting component and the second cutting component at a predetermined speed.
32. The tissue cutting system of embodiment 30, further comprising a reservoir configured to hold the tissue fragments, wherein a portion of the tissue holder protrudes into an internal space of the reservoir.
33. The tissue cutting system of embodiment 30, wherein the oscillator includes a voice coil actuator and wherein the oscillator is configured to control a frequency and an amplitude of movement for the first cutting component and the second cutting component.
34. A tissue cutting system for producing tissue fragments from a tissue sample, the tissue cutting system comprising:
   a cutting component configured to cut the tissue sample into tissue fragments of a defined size,
   a reservoir configured to collect the tissue fragments; and
   a filter assembly attachable to the reservoir, wherein the filter assembly is configured to retain tissue fragments larger than the defined size.
35. The tissue cutting system of embodiment 34, further comprising a connector coupled to the filter assembly and the reservoir, wherein the connector includes a valve configured to be adjusted between an open position and a closed position, wherein during the cutting operation the valve is in the closed position and wherein after the cutting operation the valve is in the open position, thereby allowing the contents of the reservoir to flow into the filter assembly.
36. The tissue cutting system of embodiment 35, further comprising a fluidic drive mechanism coupled to the connector and configured to drive the contents of the reservoir into the filter assembly.
37. The tissue cutting system of embodiment 34, wherein filter assembly includes a first filter unit with a first pore size configured to retain unwanted tissue fragments larger than the specified size, while allowing tissue fragments of the specified size to flow through; and a second filter unit with a second pore size connected in series to the first filter unit and configured to retain tissue fragments of the specified size.
38. The tissue cutting system of embodiment 34, further comprises a flushing mechanism configured to supply a wash buffer at a desired flow rate for washing the at least one filter unit.
39. The tissue cutting system of embodiment 34, further comprising a mechanical agitator for agitating the at least one filter unit.
40. A method of cutting a tissue sample using a tissue cutting system with a tissue holder including a hollow cavity, a first cutting component; and a second cutting component; the method comprising:
   a. preparing a tissue sample for cutting by positioning the tissue sample within the hollow cavity of the tissue holder;
   b. positioning the tissue holder in a first relative orientation;
   c. creating relative motion between the first cutting component and the tissue holder to make scoring cuts in the tissue sample in a first dimension;
   d. after step c, rotating the tissue holder by an angle to a second relative orientation;
   e. creating relative motion between the first cutting component and the tissue holder to make scoring cuts in the tissue sample in a second dimension, thereby producing a scored tissue sample;
   f. exposing a portion of the scored tissue sample through an opening at a distal end of the tissue holder;
   g. moving an exposed portion of the scored tissue sample across the second cutting component, wherein the exposed portion of the scored tissue sample is cut in a third dimension to produce tissue fragments.
41. The method of embodiment 40, wherein the angle is 90 degrees.
42. The method of embodiment 40, comprising repeating the steps from (b) to (g) wherein the entire tissue sample is cut into tissue fragments.
43. The method of embodiment 40, wherein the tissue holder further comprises a sacrificial tissue-support and wherein preparing the tissue sample for cutting further comprises positioning the tissue sample on the sacrificial tissue-support and encasing the tissue sample supported on the sacrificial tissue-support with an encapsulant.
44. The method of embodiment 40, further comprising i) moving the tissue holder to a plurality of positions while in the first relative orientation, and further moving the tissue holder towards and away from the first cutting component at each of the plurality of positions, thereby causing the first cutting component to make scoring cuts in the first dimension; and ii) moving the tissue holder to the plurality of positions while in the second relative orientation, and further moving the tissue holder towards and away from the first cutting component at each of the plurality of positions, thereby causing the first cutting component to make scoring cuts in the second dimension.
45. The method of embodiment 40, wherein the each of the plurality of positions are in a horizontal plane.
46. The method of embodiment 40, wherein the each of plurality of positions are in a vertical plane.
47. The method of embodiment 40, wherein the tissue cutting system further comprises a reservoir filled with a fluid material, and wherein during steps (b) to (g) the tissue holder containing the tissue sample is submerged within the fluid material and the tissue fragments are collected in the reservoir.
48. A method of preparing a live tissue sample for cutting comprising:
   (a) providing a tissue holder including a hollow cavity, an opening at a distal end, and a sacrificial tissue-support;
   (b) positioning the live tissue sample on a portion of the sacrificial tissue-support, wherein said portion of the sacrificial tissue-support is exposed out of the hollow cavity through the opening at the distal end of the tissue holder;
   (c) coupling an encapsulant-reservoir to the opening at the distal end of the tissue holder, the encapsulant-reservoir includes an internal volume filled with an encapsulant precursor, wherein the sacrificial tissue-support supporting the live tissue sample extends into the internal volume of the encapsulant-reservoir filled with the encapsulant precursor; and
   (d) retracting the sacrificial tissue-support supporting the live tissue sample into the hollow cavity of the tissue holder, wherein the encapsulant precursor is drawn into the hollow cavity along with the live tissue sample.
49. The method of embodiment 48, further comprising subjecting the encapsulant precursor to gelation conditions to produce an encapsulant after step (d), wherein the live tissue sample is encased by the encapsulant; and detaching the encapsulant reservoir from the tissue holder.
50. A kit for preparing a tissue sample for cutting, the kit comprising:
   a. a tissue holder comprising a hollow cavity, an opening at a distal end, and a sacrificial tissue-support, wherein the sacrificial tissue-support is configured to support the tissue sample, wherein the sacrificial tissue-support is configured to be move relative to the hollow cavity, and wherein the sacrificial tissue-support is configured to be cut; and
   b. an encapsulant-reservoir containing an encapsulant precursor, wherein the encapsulant reservoir is configured to be detachably coupled to the opening at the distal end of the tissue holder thereby permitting the flow of the encapsulant precursor from the encapsulant-reservoir into the hollow cavity of the tissue holder.
51. The kit of embodiment 50, wherein the tissue holder includes a sidewall with a slit, wherein a width of the slit is between 1 mm and 10 mm and wherein a depth of the slit is between 50 µm and 1000 µm.
52. The kit of embodiment 50, wherein the tissue holder includes a sidewall with a plurality of slits, wherein two adjacent slits of the plurality of slits are positioned apart within a range of 300 µm to 2000 µm.
53. The kit of embodiment 50, wherein the sacrificial tissue-support includes a non-flat surface with a groove.
54. The kit of embodiment 50, wherein the sacrificial tissue-support includes a non-flat surface with a plurality of grooves.
55. A cutting assembly comprising:
   a mount including
      a first surface;
      a first mount surface that intersects the first surface at a first angle, wherein the first angle is 90 degrees;
      a second mount surface that intersects the first surface at a second angle,
   wherein the second angle is within a range of 1 degree to 20 degrees;
   a first cutting component coupled to the first mount; and
   a second cutting component coupled to the second mount surface.
56. The cutting assembly of embodiment 55, wherein the second angle is 12.5 degrees.
57. The cutting assembly of embodiment 55, wherein the mount further includes
   a second surface extending parallel to the first surface, the second surface intersects the first mount surface at 90 degrees;
   a third surface extending parallel to the first mount surface; the third surface intersects the second surface at 90 degrees;
   a fourth surface extending parallel to the first surface, the fourth surface intersects the third surface at 90 degrees.
   a notch at least partially formed by the second mount surface and the fourth surface.
58. The cutting assembly of embodiment 55, wherein the mount includes a fluid input port, an outlet, and a passageway extending between the fluid input port and the outlet.
59. The cutting assembly of embodiment 58, wherein the passageway extends parallel to the first surface.
60. The cutting assembly of embodiment 58, further including a plug positioned within the outlet, the plug including a plurality of pores.
61. The cutting assembly of embodiment 55, further including a first seat extending from the first mount surface and a second seat extending from the second mount surface; wherein the first cutting component abuts the first seat and the second cutting component abuts the second seat.
62. A method of cutting a tissue sample using a tissue cutting system with a cutting component; the method comprising:
   moving the cutting component cyclically at a constant velocity in a first direction and at a constant velocity in a second direction opposite the first direction;
   moving the tissue sample toward the cutting component when the cutting component is moving at the constant velocity in the first direction or the second direction.
63. The method of embodiment 62, wherein moving the tissue sample toward the cutting component creates scoring cuts in the tissue sample.
64. The method of embodiment 62, wherein moving the tissue sample toward the cutting component creates tissue fragments.
65. The method of embodiment 62, wherein moving the tissue sample toward the cutting component includes rotating the tissue sample relative to the cutting component.

## Claims

1. A cutting assembly (50) comprising:
a mount (54) including
a first surface (56);
a first mount surface (58) that intersects the first surface (56) at a first angle (60), wherein the first angle (60) is 90 degrees;
a second mount surface (62) that intersects the first surface (56) at a second angle (64), wherein the second angle is within a range of 1 degree to 20 degrees;
a first cutting component (100) coupled to the first mount (54); and
a second cutting component (200) coupled to the second mount surface (62).

2. The cutting assembly of claim 1, wherein the second angle (64) is 12.5 degrees.

3. The cutting assembly of claim 1, wherein the mount (54) further includes
a second surface (70) extending parallel to the first surface (56), the second surface (70) intersects the first mount surface (58) at 90 degrees;
a third surface (72) extending parallel to the first mount surface (58); the third surface (72) intersects the second surface (70) at 90 degrees;
a fourth surface (74) extending parallel to the first surface (56), the fourth surface (74) intersects the third surface (72) at 90 degrees;
a notch (82) at least partially formed by the second mount surface (62) and the fourth surface (74).

4. The cutting assembly of claim 1, wherein the mount (54) includes a fluid input port (84), an outlet (86), and a passageway (88) extending between the fluid input port (84) and the outlet (86).

5. The cutting assembly of claim 4, wherein the passageway (88) extends parallel to the first surface (56).

6. The cutting assembly of claim 4, further including a plug positioned within the outlet (86), the plug including a plurality of pores.

7. The cutting assembly of claim 1, further including a first seat (66) extending from the first mount surface (58) and a second seat (68) extending from the second mount surface (62); wherein the first cutting component (100) abuts the first seat (66) and the second cutting component (200) abuts the second seat (68).

8. A method of cutting a tissue sample (300) using a tissue cutting system (10) with a cutting component (100); the method comprising:
moving the cutting component (100) cyclically at a constant velocity in a first direction and at a constant velocity in a second direction opposite the first direction;
moving the tissue sample (300) toward the cutting component (100) when the cutting component (100) is moving at the constant velocity in the first direction or the second direction.

9. The method of claim 8, wherein moving the tissue sample (300) toward the cutting component (100) creates scoring cuts in the tissue sample (300).

10. The method of claim 8, wherein moving the tissue sample toward the cutting component creates tissue fragments.

11. The method of claim 8, wherein moving the tissue sample toward the cutting component includes rotating the tissue sample (300) relative to the cutting component (100).
